# EUROPEAN PATENT SPECIFICATION

(11) **EP 3 003 363 B1**
(45) Date of publication and mention of the grant of the patent: **19.08.2020**
(21) Application number: 14727541.6
(22) Date of filing: 03.06.2014
(51) Int. Cl.: A61K 47/64

(54) **IMMUNOGENIC COMPOSITION FOR USE IN THERAPY**
IMMUNOGENE ZUSAMMENSETZUNG ZUR VERWENDUNG IN DER THERAPIE
COMPOSITION IMMUNOGÈNE POUR L'UTILISATION EN THÉRAPIE

(30) Priority: 05.06.2013 GB 201310008
(43) Date of publication of application: 13.04.2016
(73) Proprietor: GlaxoSmithKline Biologicals S.A., 1330 Rixensart (BE)
(72) Inventor: BIEMANS, Ralph Leon, 1330 Rixensart (BE); BOUTRIAU, Dominique, 1330 Rixensart (BE); DENOEL, Philippe, 1330 Rixensart (BE); DUVIVIER, Pierre, 1330 Rixensart (BE); GORAJ, Carine, 1330 Rixensart (BE)
(74) Representative: Johnston, Caroline Louise
(86) International application number: PCT/EP2014/061424
(87) International publication number: WO 2014/195280

(56) References cited:
- WO-A1-2010/151544
- WO-A2-2007/113223
- WO-A2-2012/003474
- DAUM ROBERT S ET AL: "Progress Toward a Staphylococcus aureus Vaccine", CLINICAL INFECTIOUS DISEASES, vol. 54, no. 4, February 2012 (2012-02), pages 560-567, XP002728207,

## Description

### Technical Field

The present invention relates to the field of Staphylococcal immunogenic compositions and vaccines, their manufacture and the use of such compositions in medicine. More particularly, it relates to the use of conjugates made of a capsular saccharide from S. *aureus,* conjugated to a carrier protein. Such conjugates may be combined with selected staphylococcal protein antigens to form multivalent compositions.

### Background

*Staphylococcus aureus* (*S. aureus*) are commensal, Gram-positive bacteria which colonize the nares, axilla, pharynx and other mucosal and skin surfaces of about 30% of human subjects. *S. aureus* is estimated to be responsible for 20-25% of all healthcare associated infections (Wisplinghoff et al Clin Infect. Dis. 2004; 39; 309-317), resulting in three times the length of hospital stay and a 5-fold higher risk of in-hospital death for infected patients compared to patients without such infections (Noskin et al Arch. Intern. Med. 2005; 165; 1756-1761). *S. aureus* infections can be associated with in-hospital mortality rates of up to 25%. Historically, *S. aureus* has been associated mainly with nosocomial infections. The seriousness of such infections has increased with the recent dramatic increase in *S. aureus* infection associated with antibiotic resistance. *Staphylococcus aureus* is the most common cause of nosocomial infections with a significant morbidity and mortality (Romero-Vivas et al 1995, Infect. Dis. 21; 1417). It is the cause of some cases of osteomyelitis, endocarditis, septic arthritis, pneumonia, abscesses and toxic shock syndrome.

Passive immunotherapy involving administration of polyclonal antisera against staphylococcal antigens has been investigated (WO 00/15238, WO 00/12132) as well as immunotherapy using a monoclonal antibody against lipoteichoic acid (WO 98/57994). However as yet, none have been licensed for use. Several immunotherapy candidates failed to show efficacy in humans. These include; *Altastaph* (Nabi Biopharmaceuticals) containing CP5 and CP8 antibodies purified from subjects vaccinated with *StaphVAX™* (investigational vaccine developed and trademarked by Nabi Biopharmaceuticals, Rockville, MD, USA); *Veronate* (Inhibitex), polyclonal antibodies targeting *S. aureus* clumping factor A (ClfA) and *S. epidermidis* adhesion SdrG; *Aurexis* (Tefibazumab, Inhibitex), monoclonal antibodies targetting ClfA; *Aurograb* (NeuTec Pharma), single chain antibodies against an ATP-binding cassette transporter; and Pagibaximab (Biosynexus), a monoclonal anti-lipoteichoic acid antibody (Dejonge et al J. Paediatrics 2007; 151; 260-265, Rupp et al Antimicrob. Agents Chemother. 2007; 51; 4249-4254).

An alternative approach would be use of active vaccination to generate a polyclonal immune response against staphylococci. One approach reported in WO 03/61558 uses conjugates of *S. aureus* Type 5 and Type 8 capsular polysaccharides conjugated to Pseudomonas exoprotein A (StaphVAX - Nabi Biopharmaceuticals). A further approach used a *S. aureus* IsdB protein (V710 - Merck & Co) but failed to demonstrate efficacy (Fowler et al 2013; JAMA 309; 1368-1378).

There are many problems associated with the development of a vaccine against *S. aureus* infection. The failure of vaccines relying on a single component (capsular polysaccharide or the IsdB protein) suggests that a more complex vaccine containing multiple components may be required to induce protective immunity. However, combining different antigens in an immunogenic composition can lead to interference occurring in the composition (Skurnik et al (2010) J. Clin. Invest. 120; 3220-3233). The identification of components to combine in a multivalent composition is therefore not straight forward. There remains a need to develop an effective vaccine against staphylococcal infection, especially in view of increasing frequency of multidrug resistant strains.

In the case of immunising against nosocomial staphylococcal infection, immunisation may often take place a short time only before hospitalisation or surgery or placement of an indwelling catheter. It would therefore be advantageous to achieve high levels of immunity with a single immunisation. The use of lower doses of conjugate also has advantages of relative efficiency of vaccine production and associated economic benefits.

Accordingly there is provided a disclosure of a method of immunising against *Staphylococcus aureus* infection comprising a step of administering to a human patient a single dose of an immunogenic composition comprising a *Staphylococcus aureus* Type 5 capsular saccharide conjugated to a carrier protein to form a *S. aureus* Type 5 capsular saccharide conjugate, wherein the *S. aureus* Type 5 capsular saccharide conjugate is administered at a saccharide dose of 3-50 µg, 5-25 µg, 3-20 µg, 3-12 µg, 5-10 µg, 7-20 µg, 7-15 µg or 8-12 µg.

In one aspect of the invention, there is provided an immunogenic composition comprising a *Staphylococcus aureus* Type 5 capsular saccharide conjugated to a carrier protein to form a *S. aureus* Type 5 capsular saccharide conjugate, wherein the *S. aureus* Type 5 capsular saccharide conjugate is administered at a saccharide dose of 3-50 µg, 5-25 µg, 3-20 µg, 3-12 µg, 5-10 µg, 7-20 µg, 7-15 µg or 8-12 µg, and a *S. aureus* Type 8 capsular saccharide conjugated to a carrier protein to form a *S. aureus* Type 8 capsular saccharide conjugate, wherein the *S. aureus* Type 8 capsular saccharide conjugate is administered at a saccharide dose of 3-50 µg, 5-25 µg, 3-20 µg, 3-12 µg, 5-10 µg, 7-20 µg, 7-15 µg or 8-12 µg, and a ClfA protein or fragment thereof wherein the ClfA protein or fragment thereof is at least 90% identical to the polypeptide sequence of any one of SEQ ID NO: 3-12 or 15-18 along the full length thereof, and an alpha toxoid for use in treatment or prevention of *Staphylococcus aureus* infection in which a human patient is immunised with a single dose of the immunogenic composition.

In a further aspect of the disclosure, there is provided an immunogenic composition comprising a *S. aureus* Type 5 capsular saccharide conjugated to a carrier protein, a S. *aureus* Type 8 capsular saccharide conjugated to a carrier protein, a ClfA protein or fragment thereof and an alpha toxoid.

In a further aspect of the disclosure, there is provided a vaccine comprising a *S. aureus* Type 5 capsular saccharide conjugated to a carrier protein, a *S. aureus* Type 8 capsular saccharide conjugated to a carrier protein, a ClfA protein or fragment thereof and an alpha toxoid and a pharmaceutically acceptable excipient.

In a further aspect of the invention, there is provided a process for making the immunogenic composition of the invention comprising the steps of a) conjugating a *S. aureus* Type 5 capsular saccharide to a carrier protein to form a *S. aureus* Type 5 capsular saccharide conjugate, b) conjugating a *S. aureus* Type 8 capsular saccharide conjugated to a carrier protein to form a *S. aureus* Type 8 capsular saccharide conjugate, and c) combining the *S. aureus* Type 5 capsular saccharide conjugate, the *S. aureus* Type 8 capsular saccharide conjugate, a ClfA protein or fragment thereof wherein the ClfA protein or fragment thereof is at least 90% identical to the polypeptide sequence of any one of SEQ ID NO: 3-12 or 15-18 along the full length thereof and an alpha toxoid to form the immunogenic composition.

### Description of Figures

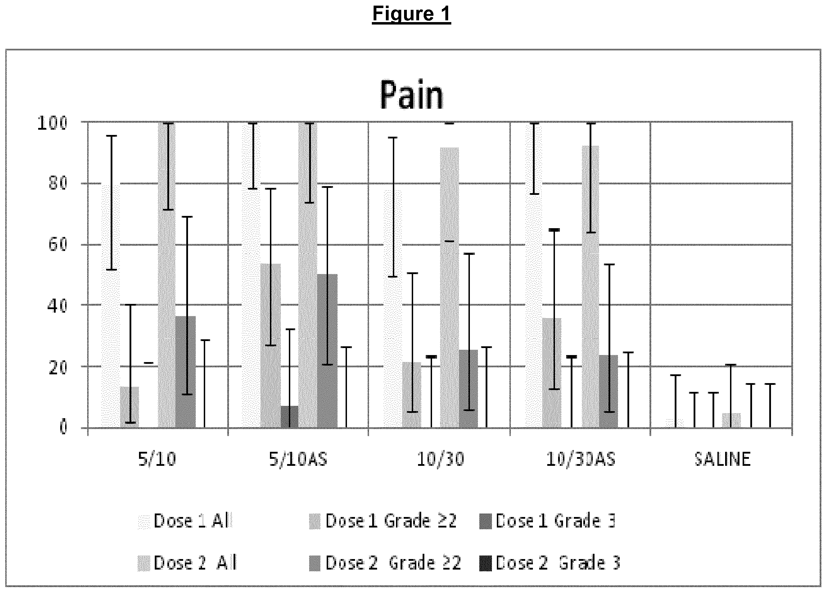

Figure 1 - Percentage of subjects experiencing pain after 1 or 2 doses of the 4C vaccine. In each formulation grouping, the first three columns provide the % of subjects experiencing pain after a single dose with the first column representing all reports of pain, the second column representing pain above or equal to grade 2 and the third column representing grade 3 pain. The 4^{th}, 5^{th} and 6^{th} columns show the same information after the second dose.

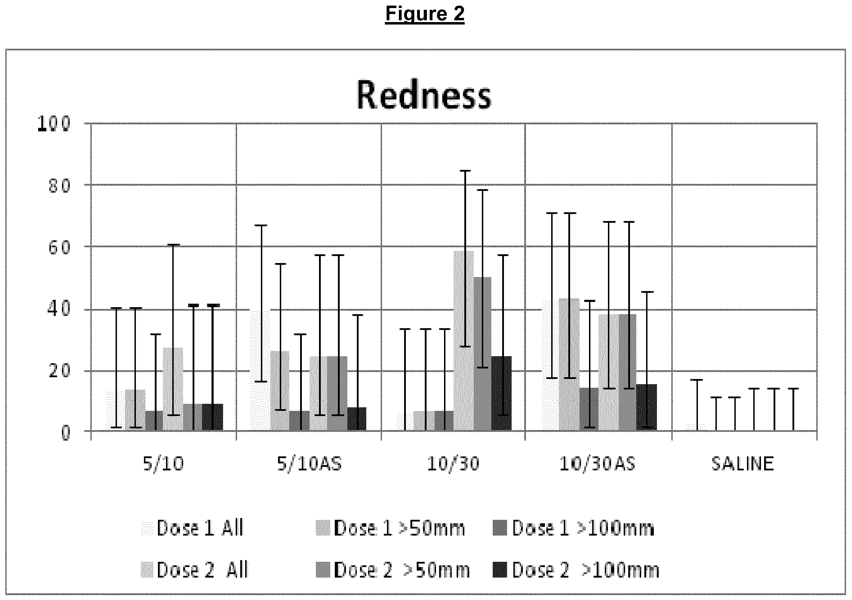

Figure 2 - Percentage of subjects experiencing redness after 1 or 2 doses of the 4C vaccine. In each formulation grouping, the first three columns provide the % of subjects experiencing redness after a single dose with the first column representing all reports of redness, the second column representing over 50mm of redness and the third column representing over 100mm of redness. The 4^{th}, 5^{th} and 6^{th} columns show the same information after the second dose.

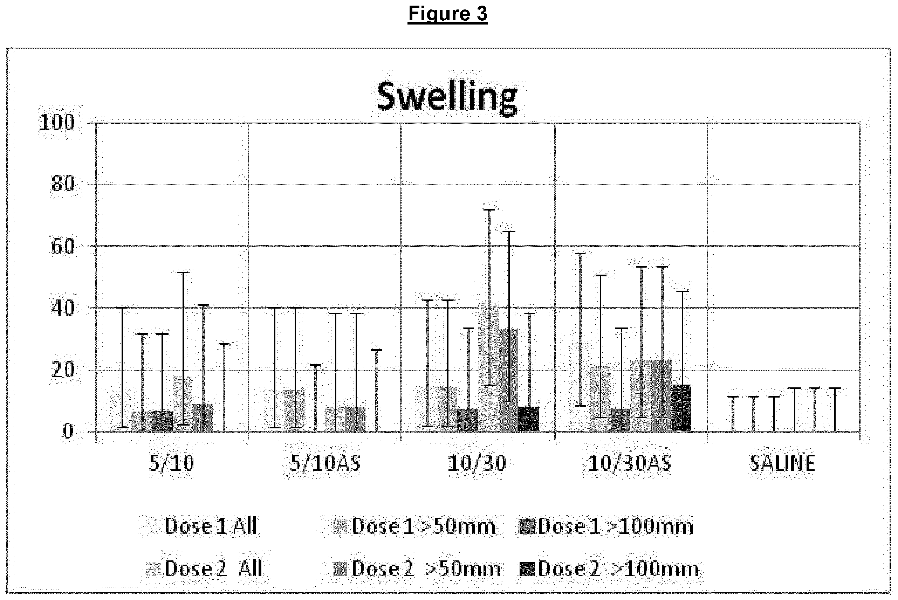

Figure 3 - Percentage of subjects experiencing swelling after 1 or 2 doses of the 4C vaccine. In each formulation grouping, the first three columns provide the % of subjects experiencing swelling after a single dose with the first column representing all reports of swelling, the second column representing over 50mm of swelling and the third column representing over 100mm of swelling. The 4^{th}, 5^{th} and 6^{th} columns show the same information after the second dose.

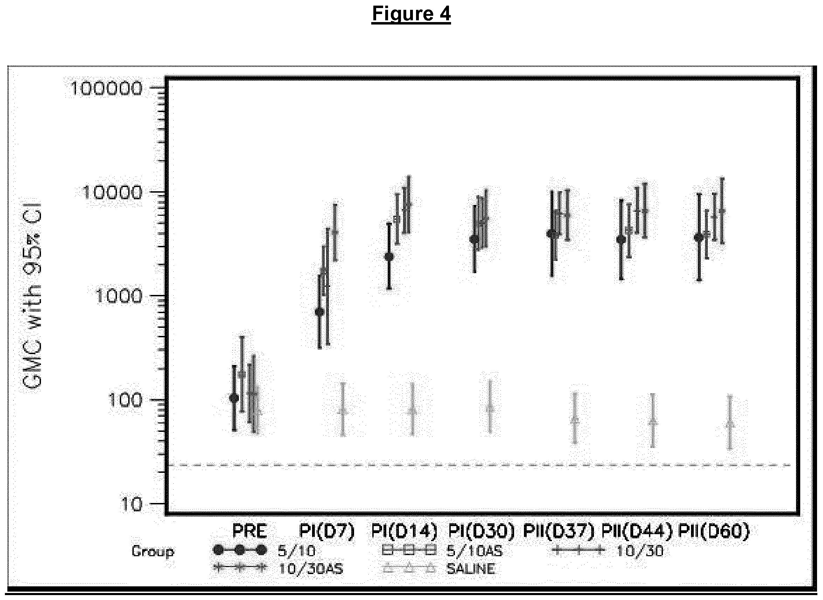

Figure 4 - Immunogenicity results for antibodies raised against *S. aureus* Type 5 capsular polysaccharide. The GMC results of a Luminex assay detecting antibodies against Type 5 capsular polysaccharide at various time points after the first and second immunisations are shown. The time points chosen are day 0 before immunisation, day 7 after one immunisation, day 14 after one immunisation, day 30 after one immunisation, day 7 after two immunisations (corresponding to day 37 on the graph), day 14 after two immunisations (corresponsing to day 44 on the graph) and day 30 after two immunisations (corresponding to day 60 on the graph). For each time point, the results are presented in the order (left to right) of, 5/10, 5/10AS, 10/30, 10/30AS and saline.

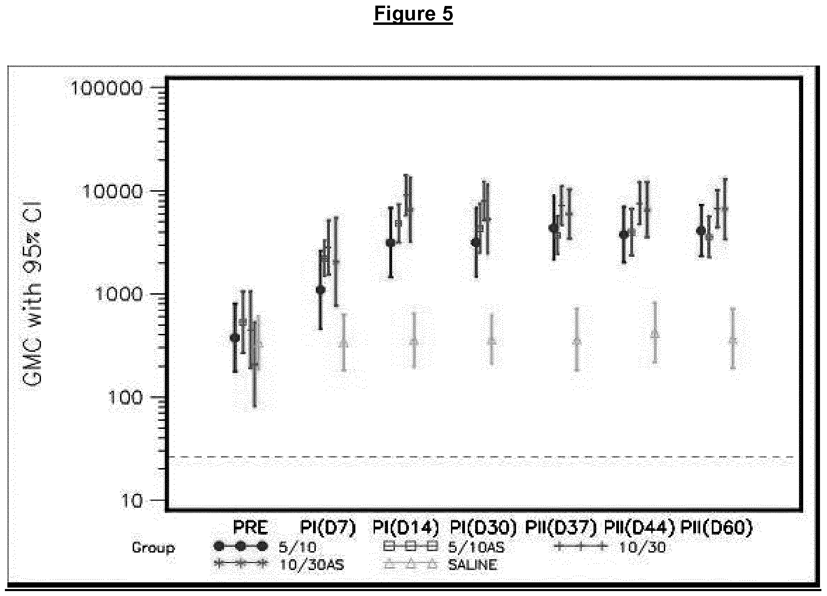

Figure 5 - Immunogenicity results for antibodies raised against *S. aureus* Type 8 capsular polysaccharide. The GMC results of a Luminex assay detecting antibodies against Type 8 capsular polysaccharide at various time points after the first and second immunisations are shown. The time points chosen are day 0 before immunisation, day 7 after one immunisation, day 14 after one immunisation, day 30 after one immunisation, day 7 after two immunisations (corresponding to day 37 on the graph), day 14 after two immunisations (corresponsing to day 44 on the graph) and day 30 after two immunisations (corresponding to day 60 on the graph). For each time point, the results are presented in the order (left to right) of, 5/10, 5/10AS, 10/30, 10/30AS and saline.

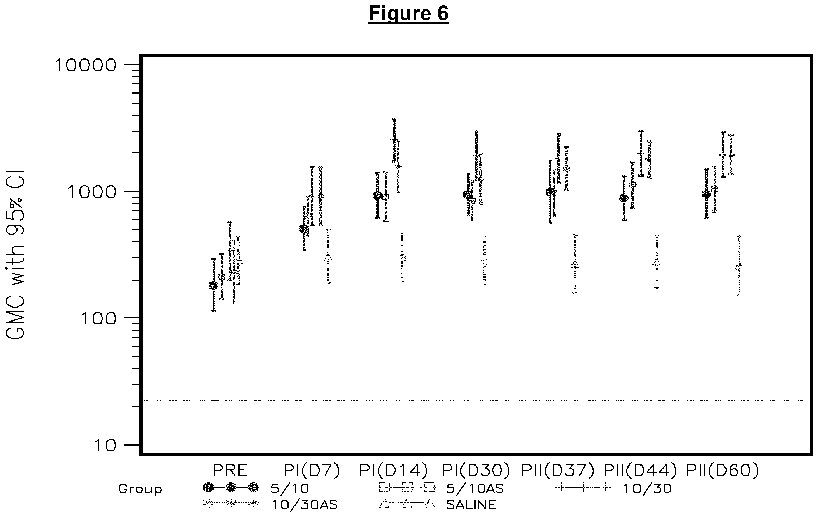

Figure 6 - Immunogenicity results for antibodies raised against *S. aureus* alpha toxoid. The GMC results of a Luminex assay detecting antibodies against alpha toxoid at various time points after the first and second immunisations are shown. The time points chosen are day 0 before immunisation, day 7 after one immunisation, day 14 after one immunisation, day 30 after one immunisation, day 7 after two immunisations (corresponding to day 37 on the graph), day 14 after two immunisations (corresponsing to day 44 on the graph) and day 30 after two immunisations (corresponding to day 60 on the graph). For each time point, the results are presented in the order (left to right) of, 5/10, 5/10AS, 10/30, 10/30AS and saline.

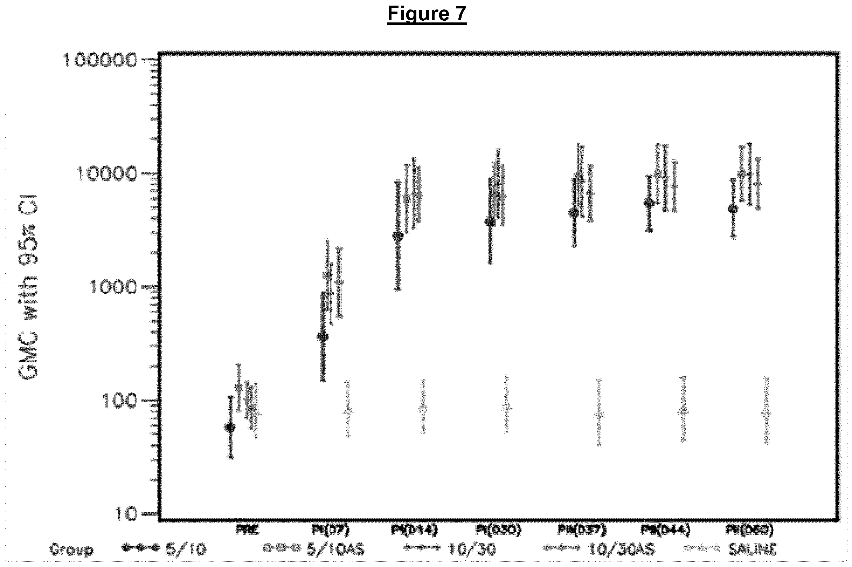

Figure 7 - Immunogenicity results for antibodies raised against *S. aureus* ClfA. The GMC results of an ELISA detecting antibodies against ClfA at various time points after the first and second immunisations are shown. The time points chosen are day 0 before immunisation, day 7 after one immunisation, day 14 after one immunisation, day 30 after one immunisation, day 7 after two immunisations (corresponding to day 37 on the graph), day 14 after two immunisations (corresponsing to day 44 on the graph) and day 30 after two immunisations (corresponding to day 60 on the graph). For each time point, the results are presented in the order (left to right) of, 5/10, 5/10AS, 10/30, 10/30AS and saline.

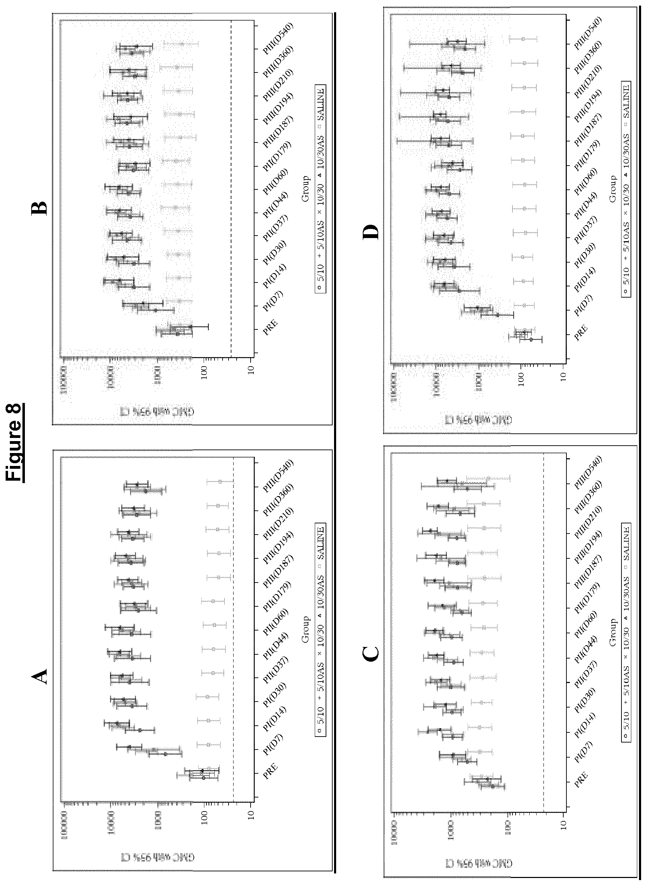

Figure 8 - Immunogenicity results for *S. aureus* Type 5 capsular polysaccharide (panel A), *S. aureus* Type 8 capsular saccharide (panel B), alpha toxoid (panel C) and ClfA (Panel D) over a longer time period of day 0 to day 540, after 1, 2 or 3 immunisations.

### Detailed description

The present disclosure provides a method of immunising against *Staphylococcus aureus* infection comprising a step of administering to a human patient a single dose of an immunogenic composition comprising a *Staphylococcus aureus* Type 5 capsular saccharide conjugated to a carrier protein to form a *S. aureus* Type 5 capsular saccharide conjugate, wherein the *S. aureus* Type 5 capsular saccharide conjugate is administered at a saccharide dose of 3-50 µg, 3-25 µg, 3-20 µg, 3-12 µg, 5-50 µg, 5-25 µg, 5-20 µg, 5-12 µg, 5-10 µg, 7-20 µg, 7-15 µg or 8-12 µg.

In an embodiment, the immunogenic composition further comprises a *S. aureus* Type 8 capsular saccharide conjugated to a carrier protein to form a *S. aureus* Type 8 capsular saccharide conjugate, wherein the *S. aureus* Type 8 capsular saccharide conjugate is administered at a saccharide dose of 3-50 µg, 3-25 µg, 3-20 µg, 3-12 µg, 5-50 µg, 5-25 µg, 5-20 µg, 5-12 µg, 5-10 µg, 7-20 µg, 7-15 µg or 8-12 µg.

In an embodiment, the same saccharide dose of *S. aureus* Type 5 capsular saccharide conjugate and *S. aureus* Type 8 capsular saccharide conjugate is present in the immunogenic composition; for example, a 4, 5, 6, 7, 8, 9 or 10 µg saccharide dose of both Type 5 and Type 8 conjugates.

Most strains of *S. aureus* that cause infection in man contain either Type 5 or Type 8 polysaccharides. Approximately 60% of human strains are Type 8 and approximately 30% are Type 5. Jones Carbohydrate Research 340, 1097-1106 (2005) used NMR spectroscopy to identify the structures of the capsular polysaccharides as:

### Type 5

→4)-β-D-ManNAcA-(1 →4)-α-L-FucNAc(3OAc)-(1 →3)-β-D-FucNAc-(1 →

### Type 8

→3)-β-D-ManNAcA(4OAc)-(1 →3)-α-L-FucNAc(1 →3)-α-D-FucNAc(1 →

Polysaccharides may be extracted from the appropriate strain of *S. aureus* using methods well known to the skilled man, for instance as described in US6294177, WO 11/41003, WO 11/51917 or Infection and Immunity (1990) 58(7); 2367. For example, ATCC 12902 is a Type 5 *S. aureus* strain and ATCC 12605 is a Type 8 *S. aureus* strain.

Polysaccharides are of native size or alternatively may be reduced in size, for instance by microfluidisation, ultrasonic irradiation or by chemical treatment such as exposure to pH 5.0-3.0. The invention also covers oligosaccharides derived from the Type 5 and 8 polysaccharides from *S. aureus.* In an embodiment the *S. aureus* Type 5 capsular saccharide has a molecular weight of over 25kDa, 30kDa, 40kDa, 50kDa, 60kDa, 70kDa, 80kDa or 90kDa or between 25-125kDa, 90-125kDa, 30-100kDa, 35-75KDa or 40-70kDa. In an embodiment the *S. aureus* Type 8 capsular saccharide has a molecular weight of over 25kDa, 30kDa, 40kDa, 50kDa, 60kDa, 70kDa, 80kDa or 90kDa or between 25-125kDa, 90-125kDa, 30-100kDa, 35-75KDa or 40-70kDa.

In an embodiment, the carrier protein to which the Type 5 and/or Type 8 capsular saccharide is conjugated is selected from the group consisting of tetanus toxoid, diphtheria toxoid, CRM197, alpha toxoid, ClfA, and *Pseudomonas aeruginosa* exoprotein A.

The Type 5 and/or 8 capsular polysaccharide or oligosaccharides included in the immunogenic composition of the invention are O-acetylated. In an embodiment, the degree of O-acetylation of Type 5 capsular polysaccharide or oligosaccharide is 50-100%, 60-100%, 70-100%, 80-100%, 90-100%, 50-90%, 60-90%, 70-90%, 70-80% or 80-90%. In an embodiment, the degree of O-acetylation of Type 8 capsular polysaccharide or oligosaccharide is 10-100%, 20-100%, 30-100%, 40-100%, 50-100%, 60-100%, 70-100%, 80-100%, 90-100%, 50-90%, 60-90%, 70-90%, 70-80% or 80-90%. In an embodiment, the degree of O-acetylation of Type 5 and Type 8 capsular polysaccharides or oligosaccharides is 10-100%, 20-100%, 30-100%, 40-100%, 50-100%. 60-100%, 70-100%, 80-100%, 90-100%, 50-90%, 60-90%, 70-90%, 70-80% or 80-90%. In an embodiment, the Type 5 and/or Type 8 capsular saccharides are 80-100% or 100% O-acetylated.

The degree of O-acetylation of the polysaccharide or oligosaccharide can be determined by any method known in the art, for example, by proton NMR (Lemercinier and Jones 1996, Carbohydrate Research 296; 83-96, Jones and Lemercinier 2002, J Pharmaceutical and Biomedical analysis 30; 1233-1247, WO 05/033148 or WO 00/56357). A further commonly used method is that described by Hestrin (1949) J. Biol. Chem. 180; 249-261.

O-acetyl groups can be removed by hydrolysis, for example by treatment with a base such as anhydrous hydrazine (Konadu et al 1994; Infect. Immun. 62; 5048-5054) or treatment with 0.1N NaOH for 1-8 hours. In order to maintain high levels of O-acetylation on Type 5 and/or 8 polysaccharide or oligosaccharide, treatments which would lead to hydrolysis of the O-acetyl groups are minimised. For example treatment at extremes of pH are minimised.

Amongst the problems associated with the use of polysaccharides in vaccination, is the fact that polysaccharides *per se* are poor immunogens. Strategies, which have been designed to overcome this lack of immunogenicity, include the linking of the polysaccharide to large protein carriers, which provide bystander T-cell help. In an embodiment, the polysaccharides utilised in the invention are linked to a protein carrier which provide bystander T -cell help. Examples of these carriers which may be used for coupling to polysaccharide or oligosaccharide immunogens include the Diphtheria and Tetanus toxoids (DT, DT Crm197 and TT), Keyhole Limpet Haemocyanin (KLH), *Pseudomonas aeruginosa* exoprotein A (rEPA) and the purified protein derivative of Tuberculin (PPD), protein D from *Haemophilus influenzae,* pneumolysin or fragments of any of the above. Fragments suitable for use include fragments encompassing T-helper epitopes. In particular protein D fragment will optionally contain the N-terminal 1/3 of the protein. Protein D is an IgD-binding protein from *Haemophilus influenzae* (EP 0 594 610 B1).

A new carrier protein that would be particularly advantageous to use in the context of a staphylococcal vaccine is staphylococcal alpha toxoid. The native form may be conjugated to a polysaccharide since the process of conjugation reduces toxicity. Optionally a genetically detoxified alpha toxin such as the His35Leu or His35Arg variants are used as carriers since residual toxicity is lower. Alternatively the alpha toxin is chemically detoxified by treatment with a cross-linking reagent, formaldehyde or glutaraldehyde. The process of conjugation is an alternative chemical treatment which detoxifies alpha toxin. A genetically detoxified alpha toxin is optionally chemically detoxified, optionally by treatment with a cross-linking reagent, formaldehyde or glutaraldehyde to further reduce toxicity.

The polysaccharides may be linked to the carrier protein(s) by any known method (for example, by Likhite, U.S. Patent 4,372,945 by Armor et al., U.S. Patent 4,474,757, Anderson et al WO 10/151544, Berti et al WO 11/138636, and Jennings et al., U.S. Patent 4,356,170). Optionally, CDAP conjugation chemistry is carried out (see WO 95/08348, WO 07/113222).

In CDAP, the cyanylating reagent 1-cyano-dimethylaminopyridinium tetrafluoroborate (CDAP) is optionally used for the synthesis of polysaccharide-protein conjugates. The cyanilation reaction can be performed under relatively mild conditions, which avoids hydrolysis of the alkaline sensitive polysaccharides. This synthesis allows direct coupling to a carrier protein.

The polysaccharide may be solubilized in water or a saline solution. CDAP may be dissolved in acetonitrile and added immediately to the polysaccharide solution. The CDAP reacts with the hydroxyl groups of the polysaccharide to form a cyanate ester. After the activation step, the carrier protein is added. Amino groups of lysine react with the activated polysaccharide to form an isourea covalent link. After the coupling reaction, a large excess of glycine is then added to quench residual activated functional groups. The product is then passed through a gel permeation column to remove unreacted carrier protein and residual reagents.

In an embodiment, the *S. aureus* Type 5 capsular saccharide and/or the *S. aureus* Type 8 capsular saccharide is directly conjugated to the carrier protein. However, the disclosure also encompasses conjugates where the Type 5 and/or 8 capsular saccharides are conjugated through a linker, for example an ADH linker.

In an embodiment, the *S. aureus* Type 5 capsular saccharide and/or the *S. aureus* Type 8 capsular saccharide is conjugated using a cyanylating reagent, for example CDAP. Alternatively, other conjugation processes such as reductive amination or carbodiimide (for example EDAC) chemistry.

In an embodiment, the ratio of polysaccharide to protein in the *S. aureus* Type 5 capsular saccharide conjugate is between 1:5 and 5:1 (w:w), 1:1 and 1:5 (w/w), 1:2 and 1:5 (w/w), 1:3 and 1:5 (w/w) 1:2 and 2:1 (w/w) or 1:1 and 1:2 (w/w). In an embodiment, the ratio of polysaccharide to protein in the *S. aureus* Type 8 capsular saccharide conjugate is between 1:5 and 5:1 (w:w), 1:1 and 1:5 (w/w), 1:2 and 1:5 (w/w), 1:3 and 1:5 (w/w) 1:2 and 2:1 (w/w) or 1:1 and 1:2 (w/w).

Clumping factor A (ClfA) has been identified as a *S. aureus* fibrinogen binding protein (US6008341) and has been identified as a potential carrier protein for polysaccharides which could be used to immunise against staphylococcal infection (WO 04/80490). ClfA is a surface located protein and is an important virulence factor due to its property of binding to fibrinogen and contributing to the adhesion of *S. aureus.* ClfA contains a fibrinogen binding region. This region, known as the A domain is located towards the N-terminus of ClfA and comprises three separately folded subdomains known as N1, N2 and N3. The A domain is followed by a serine-aspartate repeat region and a cell wall and membrane spanning region which contains the LPXTG motif for sortase-promoted anchoring to the cell wall. ClfA binds to the C-terminus of the γ-chain of fibrinogen, and is thereby able to induce clumping of bacteria in fibrinogen solution (McDevitt et al (1997) Eur. J. Biochem. 247; 416-424). Amino acid residues 221-559 of ClfA correspond to the N2-N3 region which retains fibrinogen binding. Fragments containing amino acids 221-559 of ClfA are preferred fragments. Amino acid residues 532 to 538 correspond to the latching peptide region of ClfA. Each subdomain comprises nine β-strands that form a novel IgG-type fold. The fibrinogen γ-chain peptide binding site in ClfA is located in a hydrophobic groove at the junction between N2 and N3.

Recently, amino acids P336 and Y338 of ClfA have been recognised as fibrinogen binding sites, mutation of which led to the loss of fibrinogen binding (Josefsson et al 2008, PLOS One volume 3, Issue 5, page 1-7). SEQ ID NO: 8-12, 17 and 18 contain point mutations at positions 336 and 338. The loss of fibrinogen binding in these variants led to an increased ability to protect against septic death in immunised mice, leading to the conclusion that the vaccine potential of recombinant ClfA is improved by removing its ability to bind fibrinogen (WO 09/95453). However, variants with point mutations at only one of Y256, P336, Y338 or K389 also lose their ability to bind fibrinogen (Deivanayagam et al EMBO J, 21; 6660-6672 (2002)). These single point mutations are expected to show similarly improved immunogenicity thus single mutations may also be used in the disclosure. In an embodiment, the immunogenic composition further comprises a ClfA protein or fragment thereof, optionally recombinant, isolated or purified.

In an embodiment, the ClfA protein is at least 80%, 85%, 90%, 93%, 95%, 96%, 97%, 98%, 99% or 100% identical to the polypeptide sequence of SEQ ID NO:3, 4, 5, 6 or 7 or 8-12 along the entire length of thereof.

"Identity," as known in the art, is a relationship between two or more polypeptide sequences or two or more polynucleotide sequences, as the case may be, as determined by comparing the sequences. In the art, "identity" also means the degree of sequence relatedness between polypeptide or polynucleotide sequences, as the case may be, as determined by the match between strings of such sequences. "Identity" can be readily calculated by known methods, including but not limited to those described in (Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part l, Griffin, A.M., and Griffin, H.G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48: 1073 (1988). Methods to determine identity are designed to give the largest match between the sequences tested. Moreover, methods to determine identity are codified in publicly available computer programs. Computer program methods to determine identity between two sequences include, but are not limited to, the GAP program in the GCG program package (Devereux, J., et al., Nucleic Acids Research 12(1): 387 (1984)), BLASTP, BLASTN (Altschul, S.F. et al., J. Molec. Biol. 215: 403-410 (1990)), and FASTA (Pearson and Lipman Proc. Natl. Acad. Sci. USA 85; 2444-2448 (1988)). The BLAST family of programs is publicly available from NCBI and other sources (BLAST Manual, Altschul, S., et al., NCBI NLM NIH Bethesda, MD 20894; Altschul, S., et al., J. Mol. Biol. 215: 403-410 (1990)). The well known Smith Waterman algorithm may also be used to determine identity.

Parameters for polypeptide sequence comparison include the following:
Algorithm: Needleman and Wunsch, J. Mol Biol. 48: 443-453 (1970)
Comparison matrix: BLOSSUM62 from Henikoff and Henikoff,
Proc. Natl. Acad. Sci. USA. 89:10915-10919 (1992)
Gap Penalty: 8
Gap Length Penalty: 2

A program useful with these parameters is publicly available as the "gap" program from Genetics Computer Group, Madison Wl. The aforementioned parameters are the default parameters for peptide comparisons (along with no penalty for end gaps).

Parameters for polynucleotide comparison include the following:
Algorithm: Needleman and Wunsch, J. Mol Biol. 48: 443-453 (1970)
Comparison matrix: matches = +10, mismatch = 0
Gap Penalty: 50
Gap Length Penalty: 3
Available as: The "gap" program from Genetics Computer Group, Madison Wl. These are the default parameters for nucleic acid comparisons.

Where a protein is specifically mentioned herein, it is optionally a reference to a native or recombinant, full-length protein or optionally a mature protein in which any signal sequence has been removed. The protein may be isolated directly from the staphylococcal strain or produced by recombinant DNA techniques. Immunogenic fragments of the protein may be incorporated into the immunogenic composition of the disclosure. These are fragments comprising at least 10 amino acids, at least 20 amino acids, at least 30 amino acids, at least 40 amino acids, at least 50 amino acids or at least 100 amino acids, taken contiguously from the amino acid sequence of the protein. In addition, such immunogenic fragments are typically immunologically reactive with antibodies generated against the Staphylococcal proteins or with antibodies generated by infection of a mammalian host with Staphylococci or contain T cell epitopes. In an embodiment, immunogenic fragments also includes fragments that when administered at an effective dose, (either alone or as a hapten bound to a carrier), elicit a protective immune response against Staphylococcal infection, optionally it is protective against *S. aureus* and/or *S*. *epidermidis* infection. Such an immunogenic fragment may include, for example, the protein lacking an N-terminal leader sequence, and/or a transmembrane domain and/or a C-terminal anchor domain. For ClfA, preferred fragments lack the SD repeat domain towards the C-terminus of ClfA (for example by using a fragment in which amino acids 555-927, 556-927, 557-927, 558-927, 559-927 or 560-927 are deleted). For ClfA and alpha toxoid, preferred fragments have the signal peptide removed to form the mature protein, optionally with an initial methionine residue at the N-terminus to allow recombinant expression.

In an embodiment, immunogenic compositions of the disclosure may contain fusion proteins or fragments of ClfA. The fusion protein optionally contains heterologous sequences such as a provider of T-cell epitopes or purification tags, for example: β-galactosidase, glutathione-S-transferase, green fluorescent proteins (GFP), epitope tags such as FLAG, myc tag, poly histidine, or viral surface proteins such as influenza virus haemagglutinin, or bacterial proteins such as tetanus toxoid, diphtheria toxoid, CRM197. The fusion protein may be present in the immunogenic composition of the disclosure as a free protein or it may be a carrier protein linked to a saccharide.

In an embodiment, the disclosure also provides an immunogenic fragment of the ClfA protein that is, a contiguous portion of the ClfA polypeptide which has the same or substantially the same immunogenic activity as the polypeptide comprising the polypeptide sequence of SEQ ID NO:3. That is to say, the fragment (if necessary when coupled to a carrier) is capable of raising an immune response which recognises ClfA polypeptide. Such an immunogenic fragment may include, for example, the ClfA polypeptide lacking an N-terminal leader sequence, and/or the SD repeat domain toward the C-terminus of ClfA. In a preferred embodiment the immunogenic fragment of ClfA comprises substantially all of the fibrinogen binding domain and has at least 85% identity, preferably at least 90% identity, more preferably at least 95% identity, most preferably at least 97-99% identity or 100% identity, to the amino acid sequence of any one of SEQ ID NO:4-12 over the entire length of said sequence.

Fragments may be "free-standing," or comprised within a larger polypeptide of which they form a part or region, most preferably as a single continuous region in a single larger polypeptide. Further fragments of ClfA include an isolated polypeptide comprising an amino acid sequence having at least 15, 20, 30, 40, 50 or 100 contiguous amino acids from the amino acid sequence of SEQ ID NO:3.

In an embodiment, the ClfA protein is a fragment of ClfA comprising the N1 domain, the N2 domain, the N3 domain, the N1 and N2 domains, the N2 and N3 domains or the N1 and N2 and N3 domains. Optionally, the ClfA fragment comprises the N2 and N3 domains and has an amino acid sequence at least 80%, 85%, 90%, 95%, 97%, 98%, 99% or 100% identical to the sequence of SEQ ID NO: 6, 7, 11 or 12.

In an embodiment, the ClfA protein or fragment thereof contains an amino acid substitution, deletion or insertion which reduces or abolishes the ability of ClfA to bind to fibrinogen. In an embodiment, the ability of ClfA to bind to fibrinogen is reduced by at least 10, 20, 30, 40, 50, 60, 70, 80, 90, 95 or 99%. Such a mutation is typically in the fibrinogen binding region at the N-terminus of ClfA. The mutation is optionally an amino acid substitution at at least one, two, three or four of amino acids Ala254, Tyr256, Pro336, Tyr338, Ile387, Lys389, Tyr474, Glu526 or Val527. In an embodiment, ClfA amino acid Pro336 is mutated. In an embodiment ClfA amino acid Tyr338 is mutated. In an embodiment, both Pro336 and Tyr338 are mutated, optionally to Alanine or Serine. In an embodiment, ClfA contains two mutations with Pro336 mutated to Ser and Tyr 338 mutated to Ala.

In an embodiment, the ClfA protein or fragment is present in the immunogenic composition as an unconjugated protein. Alternatively, it is present conjugated to the *S. aureus* Type 5 capsular saccharide or to the *S. aureus* Type 8 capsular saccharide. In such cases, ClfA may act as a carrier protein and an antigen.

In an embodiment, the ClfA protein or fragment thereof is present in the immunogenic composition at a dose of 5-50, 10-30, 5-15 or 20-40 µg.

Alpha toxin is an important virulence determinant produced by most strains of *S. aureus.* It is a pore forming toxin with haemolytic activity. Antibodies against alpha toxin have been shown to neutralise the detrimental and lethal effects of alpha toxin in animal models (Adlam et al 1977 Infect. Immun. 17; 250). Human platelets, endothelial cells and mononuclear cells are susceptible to the effects of alpha toxin. In order for alpha toxin to be used in an immunogenic composition, it is typically detoxified by chemical treatment or mutation to produce alpha toxoid.

In an embodiment, the immunogenic composition comprises an alpha toxoid. Optionally the alpha toxoid has an amino acid sequence at least 90%, 95%, 96%, 97%, 98% or 99% identical to SEQ ID NO:1 or 2.

The high toxicity of alpha toxin requires that it should be detoxified before being used as an immunogen. This can be achieved by chemical treatment, for instance by treating with formaldehyde, glutaraldehyde of other cross-linking reagents or by chemically conjugating it to bacterial polysaccharides as described above.

A further way of removing toxicity is to introduce point mutations that remove toxicity while retaining the immunogenicity of the toxin. The introduction of a point mutation at amino acid 35 of alpha toxin where a histidine residue is replaced with a leucine residue results in the removal of toxicity whilst retaining immunogenicity (Menzies and Kernodle 1996; Infect. Immun. 64; 1839). Histidine 35 appears to be critical for the proper oligomerization required for pore formation and mutation of this residue leads to loss of toxicity. The modification of histidine 35 may be a substitution with Lys, Arg, Ala, Leu or Glu. Point mutation of alpha toxin at Asp24, Lys37, His48, Lys58, Asp100, Ile107, Glu111, Met113, Asp127, Asp128, Gly130, Gly134, His144, Lys147, Gln150, Asp152, Phe153, Lys154, Val169, Asn173, Arg200, Asn214, Leu219 or His259 can optionally be used to reduce toxicity.

When incorporated into immunogenic compositions of the disclosure, alpha toxoid is optionally detoxified by mutation of His 35, for example by replacing His 35 with Leu or Arg. In an alternative embodiment, alpha toxoid is detoxified by conjugation to other components of the immunogenic composition, for example to *S. aureus* Type 5 polysaccharide and/or *S. aureus* Type 8 polysaccharide. In an embodiment, the alpha toxoid is detoxified by both the introduction of a point mutation and by conjugation to *S. aureus* Type 5 polysaccharide and/or *S. aureus* Type 8 polysaccharide.

In an embodiment, the immunogenic composition comprises alpha toxoid which contains a point mutation which decreases toxicity of alpha toxin, for example at amino acid 35. The alpha toxoid optionally contains a point mutation at amino acid 35 where histidine is replaced with an arginine amino acid.

In an embodiment, the alpha toxoid is present in the immunogenic composition as an unconjugated protein. Alternatively, the alpha toxoid is conjugated to the *S. aureus* Type 5 capsular saccharide and/or to the *S. aureus* Type 8 capsular saccharide.

In an embodiment, the alpha toxoid is present in the immunogenic composition at a dose of 5-50, 10-30, 5-15 or 20-40 µg. In an embodiment, the ClfAand alpha toxoid are present at the same dose in the immunogenic composition. In an embodiment the saccharide dose of Type 5 and 8 capsular saccharide conjugates is higher than the protein dose of ClfA and alpha toxoid.

In an embodiment, the immunogenic composition of the disclosure is mixed with a pharmaceutically acceptable excipient, and optionally with an adjuvant to form a vaccine.

The vaccines of the present disclosure may be adjuvanted, particularly when intended for use in an elderly, immunocompromised or chronically ill populations (such as diabetes, end stage renal disease or other populations at high risk of staphylococcal infection) but also for use in infant populations. Suitable adjuvants include an aluminium salt such as aluminium hydroxide gel or aluminium phosphate or alum, but may also be other metal salts such as those of calcium, magnesium, iron or zinc. Oil in water emulsions, for example comprising metabolisable oil (for example squalene), emulsifying agent (for example polyoxyethylene sorbitan monooleate) and optionally a tocol (for example alpha tocopherol) are also suitable (WO 09/95453).

It is preferred that the adjuvant be selected to be a preferential inducer of a TH1 type of response. Such high levels of Th1-type cytokines tend to favour the induction of cell mediated immune responses to a given antigen, whilst high levels of Th2-type cytokines tend to favour the induction of humoral immune responses to the antigen.

The distinction of Th1 and Th2-type immune response is not absolute. In reality an individual will support an immune response which is described as being predominantly Th1 or predominantly Th2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties (Annual Review of Immunology, 7, p145-173). Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of II-4, IL-5, IL-6, IL-10. Suitable adjuvant systems which promote a predominantly Th1 response include: Monophosphoryl lipid A or a derivative thereof (or detoxified lipid A in general - see for instance WO2005107798), particularly 3-de-O-acylated monophosphoryl lipid A (3D-MPL) (for its preparation see GB 2220211 A); and a combination of monophosphoryl lipid A, preferably 3-de-O-acylated monophosphoryl lipid A, together with either an aluminum salt (for instance aluminum phosphate or aluminum hydroxide) or an oil-in-water emulsion. In such combinations, antigen and 3D-MPL are contained in the same particulate structures, allowing for more efficient delivery of antigenic and immunostimulatory signals. Studies have shown that 3D-MPL is able to further enhance the immunogenicity of an alum-adsorbed antigen [Thoelen et al. Vaccine (1998) 16:708-14; EP 689454-B1].

A further system involves the combination of a monophosphoryl lipid A and a saponin derivative, particularly the combination of QS21 and 3D-MPL as disclosed in WO 94/00153, or a less reactogenic composition where the QS21 is quenched with cholesterol as disclosed in WO 96/33739. A further adjuvant formulation involving QS21, 3D-MPL and tocopherol in an oil in water emulsion is described in WO 95/17210. In one embodiment the immunogenic composition additionally comprises a saponin, which may be QS21. The formulation may also comprise an oil in water emulsion and tocopherol (WO 95/17210). Unmethylated CpG containing oligonucleotides (WO 96/02555) and other immunomodulatory oligonucleotides (WO0226757 and WO03507822 are also preferential inducers of a TH1 response and are suitable for use in the present disclosure.

However, the inventors have found that in a clinical trial, the addition of an oil in water emulsion adjuvant did not produce an increase in immunogenicity. In view of the increased reactogenicity which can be associated with the use of adjuvant, an embodiment of the disclosure uses an unadjuvanted immunogenic composition, for example an immunogenic composition in which none of the staphylococcal components present is adsorbed to an adjuvant or an immunogenic composition in which the staphylococcal components are not mixed with an oil in water emulsion adjuvant. The staphylococcal components comprise 1, 2, 3 or 4 of a *S. aureus* Type 5 capsular saccharide conjugate, a *S. aureus* Type 8 capsular saccharide conjugate, a ClfA fragment or fragment thereof and an alpha toxoid.

A further aspect of the disclosure is a vaccine comprising the immunogenic composition described above and a pharmaceutically acceptable excipient. The vaccine preparations of the present disclosure may be used to protect or treat a human susceptible to *S. aureus* infection, by means of administering said vaccine via systemic or mucosal route. These administrations may include injection *via* the intramuscular, intraperitoneal, intradermal or subcutaneous routes; or *via* mucosal administration to the oral/alimentary, respiratory, genitourinary tracts.

Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

The vaccines of the present disclosure may be stored in solution or lyophilized. Optionally the solution is lyophilized in the presence of a sugar such as sucrose, trehalose or lactose. It is typical that they are lyophilized and extemporaneously reconstituted prior to use. Lyophilizing may result in a more stable composition (vaccine).

The disclosure also encompasses method of making the immunogenic compositions and vaccines of the disclosure. In an embodiment, the process of the disclosure, is a method to make a vaccine comprising the steps of a) conjugating a *S. aureus* Type 5 capsular saccharide to a carrier protein to form a *S. aureus* Type 5 capsular saccharide conjugate, b) conjugating a *S. aureus* Type 8 capsular saccharide conjugated to a carrier protein to form a *S. aureus* Type 8 capsular saccharide conjugate, and c) combining the *S. aureus* Type 5 capsular saccharide conjugate, the *S. aureus* Type 8 capsular saccharide conjugate, a ClfA protein or fragment thereof and an alpha toxoid to form the immunogenic composition. In an embodiment, the process comprises a further step of adding a pharmaceutically acceptable excipient.

The disclosure also encompasses method of treatment or staphylococcal infection, particularly hospital acquired nosocomial infections.

This immunogenic composition or vaccine of the disclosure is particularly advantageous to use in cases of elective surgery, particularly when the subjects are immunised with a single dose. Such patients will know the date of surgery in advance and can advantageously be inoculated in advance. In an embodiment, the subject is immunised with a single dose of the immunogenic composition of the disclosure 5-60, 6-40, 7-30 or 7-15 days before admission to hospital. In an embodiment, the subject is immunised with a single dose of the immunogenic composition of the disclosure 5-60, 6-40, 7-30 or 7-15 days before a planned hospital procedure, for example a surgical procedure such as a cardio-thoracic surgical procedure. Typically adults over 16 awaiting elective surgery are treated with the immunogenic compositions and vaccines of the disclosure. Alternatively children aged 3-16 awaiting elective surgery are treated with the immunogenic compositions and vaccines of the disclosure.

It is also possible to inoculate health care workers with the vaccine of the invention.

The vaccine preparations of the present disclosure may be used to protect or treat a human susceptible to *S. aureus* infection, by means of administering said vaccine via systemic or mucosal route. These administrations may include injection *via* the intramuscular, intraperitoneal, intradermal or subcutaneous routes; or *via* mucosal administration to the oral/alimentary, respiratory, genitourinary tracts.

An embodiment of the disclosure is a method of preventing or treating staphylococcal infection or disease comprising the step of administering the immunogenic composition or vaccine of the disclosure to a patient in need thereof.

A further embodiment of the disclosure is a use of the immunogenic composition of the invention in the manufacture of a vaccine for treatment or prevention of staphylococcal infection or disease, optionally post-surgery staphylococcal infection.

The terms "comprising", "comprise" and "comprises" herein are intended by the inventors to be optionally substitutable with the terms "consisting of", "consist of" and "consists of", respectively, in every instance. However, the terms "comprising", "comprise" and "comprises" retain their usual "open" meaning where they have not been substituted.

In order that this disclosure may be better understood, the following examples are set forth. These examples are for purposes of illustration only, and are not to be construed as limiting the scope of the invention in any manner.

### Examples

### Example 1 Sequences of proteins

SEQ ID NO:1

MKTRIVSSVTTTLLLGSILMNPVANAADSDINIKTGTTDIGSNTTVKTGDLVTYDKENGMHKKVFY
SFIDDKNHNKKLLVIRTKGTIAGQYRVYSEEGANKSGLAWPSAFKVQLQLPDNEVAQISDYYPRNS
IDTKEYMSTLTYGFNGNVTGDDTGKIGGLIGANVSIGHTLKYVQPDFKTILESPTDKKVGWKVIFN
NMVNQNWGPYDRDSWNPVYGNQLFMKTRNGSMKAADNFLDPNKASSLLSSGFSPDFATVITMDRKA
SKQQTNIDVIYERVRDDYQLHWTSTNWKGTNTKDKWIDRSSERYKIDWEKEEMTN

SEQ ID NO:2
MADSDINIKTGTTDIGSNTTVKTGDLVTYDKENGMHKKVFYSFIDDKNHNKKLLVIRTKGTIAGQY
RVYSEEGANKSGLAWPSAFKVQLQLPDNEVAQISDYYPRNSIDTKEYMSTLTYGFNGNVTGDDTGK
IGGLIGANVSIGHTLKYVQPDFKTILESPTDKKVGWKVIFNNMVNQNWGPYDRDSWNPVYGNQLFM
KTRNGSMKAADNFLDPNKASSLLSSGFSPDFATVITMDRKASKQQTNIDVIYERVRDDYQLHWTST
NWKGTNTKDKWIDRSSERYKIDWEKEEMTN

SEQ ID NO:3

MNMKKKEKHAIRKKSIGVASVLVGTLIGFGLLSSKEADASENSVTQSDSASNESKSNDSSSVSAAPKTDDTNV
SDTKTSSNTNNGETSVAQNPAQQETTQSSSTNATTEETPVTGEATTTTTNQANTPATTQSSNTNAEELVNQTS
NETTSNDTNTVSSVNSPQNSTNAENVSTTQDTSTEATPSNNESAPQSTDASNKDVVNQAVNTSAPRMRAFSLA
AVAADAPVAGTDITNQLTNVTVGIDSGTTVYPHQAGYVKLNYGFSVPNSAVKGDTFKITVPKELNLNGVTSTA
KVPPIMAGDQVLANGVIDSDGNVIYTFTDYVNTKDDVKATLTMPAYIDPENVKKTGNVTLATGIGSTTANKTV
LVDYEKYGKFYNLSIKGTIDQIDKTNNTYRQTIYVNPSGDNVIAPVLTGNLKPNTDSNALIDQQNTSIKVYKV
DNAADLSESYFVNPENFEDVTNSVNITFPNPNQYKVEFNTPDDQITTPYIVVVNGHIDPNSKGDLALRSTLYG
YNSNIIWRSMSWDNEVAFNNGSGSGDGIDKPVVPEQPDEPGEIEPIPEDSDSDPGSDSGSDSNSDSGSDSGSD
STSDSGSDSASDSDSASDSDSASDSDSASDSDSASDSDSDNDSDSDSDSDSDSDSDSDSDSDSDSDSDSDSDS
DSDSDSDSDSDSDSDSDSDSDSDSDSDSDSDSDSDSDSDSDSDSDSDSDSDSDSDSDSDSDSDSDSDSDSDSD
SDSDSDSDSDSDSDSDSDSDSDSDSDSDSDSDSDSDSDSDSASDSDSDSDSDSDSDSDSDSDSDSDSDSDSDS
DSDSESDSDSDSDSDSDSDSDSDSDSDSDSASDSDSGSDSDSSSDSDSESDSNSDSESVSNNNVVPPNSPKNGTN
ASNKNEAKDSKEPLPDTGSEDEANTSLIWGLLASIGSLLLFRRKKENKDKK

SEQ ID NO:4

MSENSVTQSDSASNESKSNDSSSVSAAPKTDDTNVSDTKTSSNTNNGETSVAQNPAQQETTQSSSTNATTEETPVTGEATT

TTTNQANTPATTQSSNTNAEELVNQTSNETTSNDTNTVSSVNSPQNSTNAENVSTTQDTSTEATPSNNESAPQSTDASNKD

VVNQAVNTSAPRMRAFSLAAVAADAPVAGTDITNQLTNVTVGIDSGTTVYPHQAGYVKLNYGFSVPNSAVKGDTFKITVPK

ELNLNGVTSTAKVPPIMAGDQVLANGVIDSDGNVIYTFTDYVNTKDDVKATLTMPAYIDPENVKKTGNVTLATGIGSTTAN

KTVLVDYEKYGKFYNLSIKGTIDQIDKTNNTYRQTIYVNPSGDNVIAPVLTGNLKPNTDSNALIDQQNTSIKVYKVDNAAD

LSESYFVNPENFEDVTNSVNITFPNPNQYKVEFNTPDDQITTPYIVVVNGHIDPNSKGDLALRSTLYGYNSNIIWRSMSWD

NEVAFNNGSGSGDGIDKPVVPEQPDEPGEIEPIPE

SEQ ID NO:5

MNMKKKEKHAIRKKSIGVASVLVGTLIGFGLLSSKEADASENSVTQSDSASNESKSNDSSSVSAAPKTDDTNV
SDTKTSSNTNNGETSVAQNPAQQETTQSSSTNATTEETPVTGEATTTTTNQANTPATTQSSNTNAEELVNQTS
NETTSNDTNTVSSVNSPQNSTNAENVSTTQDTSTEATPSNNESAPQSTDASNKDVVNQAVNTSAPRMRAFSLA
AVAADAPVAGTDITNQLTNVTVGIDSGTTVYPHQAGYVKLNYGFSVPNSAVKGDTFKITVPKELNLNGVTSTA
KVPPIMAGDQVLANGVIDSDGNVIYTFTDYVNTKDDVKATLTMPAYIDPENVKKTGNVTLATGIGSTTANKTV
LVDYEKYGKFYNLSIKGTIDQIDKTNNTYRQTIYVNPSGDNVIAPVLTGNLKPNTDSNALIDQQNTSIKVYKV
DNAADLSESYFVNPENFEDVTNSVNITFPNPNQYKVEFNTPDDQITTPYIVVVNGHIDPNSKGDLALRSTLYG
YNSNIIWRSMSWDNEVAFNNGSGSGDGIDKPVVPEQPDEPGEIEPIPE

SEQ ID NO:6

SLAAVAADAPVAGTDITNQLTNVTVGIDSGTTVYPHQAGYVKLNYGFSVPNSAVKGDTFKITVPKELNLNGVT
STAKVPPIMAGDQVLANGVIDSDGNVIYTFTDYVNTKDDVKATLTMPAYIDPENVKKTGNVTLATGIGSTTAN
KTVLVDYEKYGKFYNLSIKGTIDQIDKTNNTYRQTIYVNPSGDNVIAPVLTGNLKPNTDSNALIDQQNTSIKV
YKVDNAADLSESYFVNPENFEDVTNSVNITFPNPNQYKVEFNTPDDQITTPYIVVVNGHIDPNSKGDLALRST
LYGYNSNIIWRSMSWDNEVAFNNGSGSGDGIDKPVVPEQPDEPGEIEPIPE

SEQ ID NO:7

GTDITNQLTNVTVGIDSGTTVYPHQAGYVKLNYGFSVPNSAVKGDTFKITVPKELNLNGVTSTAKVPPIMAG
DQVLANGVIDSDGNVIYTFTDYVNTKDDVKATLTMPAYIDPENVKKTGNVTLATGIGSTTANKTVLVDYEKYG
KFYNLSIKGTIDQIDKTNNTYRQTIYVNPSGDNVIAPVLTGNLKPNTDSNALIDQQNTSIKVYKVDNAADLSE
SYFVNPENFEDVTNSVNITFPNPNQYKVEFNTPDDQITTPYIVVVNGHIDPNSKGDLALRSTLYGYNSNIIWR
SMSWDNEVAFNNGSGSGDGIDKPVVPEQPDEPGEIEPIPE

SEQ ID NO:8

MNMKKKEKHAIRKKSIGVASVLVGTLIGFGLLSSKEADASENSVTQSDSASNESKSNDSSSVSAAPKTDDTNV
SDTKTSSNTNNGETSVAQNPAQQETTQSSSTNATTEETPVTGEATTTTTNQANTPATTQSSNTNAEELVNQTS
NETTSNDTNTVSSVNSPQNSTNAENVSTTQDTSTEATPSNNESAPQSTDASNKDVVNQAVNTSAPRMRAFSLA
AVAADAPVAGTDITNQLTNVTVGIDSGTTVYPHQAGYVKLNYGFSVPNSAVKGDTFKITVPKELNLNGVTSTA
KVPPIMAGDQVLANGVIDSDGNVIYTFTDYVNTKDDVKATLTMSAAIDPENVKKTGNVTLATGIGSTTANKTV
LVDYEKYGKFYNLSIKGTIDQIDKTNNTYRQTIYVNPSGDNVIAPVLTGNLKPNTDSNALIDQQNTSIKVYKV
DNAADLSESYFVNPENFEDVTNSVNITFPNPNQYKVEFNTPDDQITTPYIVVVNGHIDPNSKGDLALRSTLYG
YNSNIIWRSMSWDNEVAFNNGSGSGDGIDKPVVPEQPDEPGEIEPIPEDSDSDPGSDSGSDSNSDSGSDSGSD
STSDSGSDSASDSDSASDSDSASDSDSASDSDSASDSDSDNDSDSDSDSDSDSDSDSDSDSDSDSDSDSDSDS
DSDSDSDSDSDSDSDSDSDSDSDSDSDSDSDSDSDSDSDSDSDSDSDSDSDSDSDSDSDSDSDSDSDSDSDSD
SDSDSDSDSDSDSDSDSDSDSDSDSDSDSDSDSDSDSDSASDSDSDSDSDSDSDSDSDSDSDSDSDSDSDSDS
DSDSESDSDSDSDSDSDSDSDSDSDSDSDSDSASDSDSGSDSDSSSDSDSESDSNSDSESVSNNNVVPPNSPKNGTN
ASNKNEAKDSKEPLPDTGSEDEANTSLIWGLLASIGSLLLFRRKKENKDKK

SEQ ID NO:9

MSENSVTQSDSASNESKSNDSSSVSAAPKTDDTNVSDTKTSSNTNNGETSVAQNPAQQETTQSSSTNATTEETPVTGEATT
TTTNQANTPATTQSSNTNAEELVNQTSNETTSNDTNTVSSVNSPQNSTNAENVSTTQDTSTEATPSNNESAPQSTDASNKD
VVNQAVNTSAPRMRAFSLAAVAADAPVAGTDITNQLTNVTVGIDSGTTVYPHQAGYVKLNYGFSVPNSAVKGDTFKITVPK
ELNLNGVTSTAKVPPIMAGDQVLANGVIDSDGNVIYTFTDYVNTKDDVKATLTMSAAIDPENVKKTGNVTLATGIGSTTAN
KTVLVDYEKYGKFYNLSIKGTIDQIDKTNNTYRQTIYVNPSGDNVIAPVLTGNLKPNTDSNALIDQQNTSIKVYKVDNAAD

LSESYFVNPENFEDVTNSVNITFPNPNQYKVEFNTPDDQITTPYIVVVNGHIDPNSKGDLALRSTLYGYNSNIIWRSMSWD
NEVAFNNGSGSGDGIDKPVVPEQPDEPGEIEPIPE

SEQ ID NO:10

MNMKKKEKHAIRKKSIGVASVLVGTLIGFGLLSSKEADASENSVTQSDSASNESKSNDSSSVSAAPKTDDTNV
SDTKTSSNTNNGETSVAQNPAQQETTQSSSTNATTEETPVTGEATTTTTNQANTPATTQSSNTNAEELVNQTS
NETTSNDTNTVSSVNSPQNSTNAENVSTTQDTSTEATPSNNESAPQSTDASNKDVVNQAVNTSAPRMRAFSLA
AVAADAPVAGTDITNQLTNVTVGIDSGTTVYPHQAGYVKLNYGFSVPNSAVKGDTFKITVPKELNLNGVTSTA
KVPPIMAGDQVLANGVIDSDGNVIYTFTDYVNTKDDVKATLTMSAAIDPENVKKTGNVTLATGIGSTTANKTV
LVDYEKYGKFYNLSIKGTIDQIDKTNNTYRQTIYVNPSGDNVIAPVLTGNLKPNTDSNALIDQQNTSIKVYKV
DNAADLSESYFVNPENFEDVTNSVNITFPNPNQYKVEFNTPDDQITTPYIVVVNGHIDPNSKGDLALRSTLYG
YNSNIIWRSMSWDNEVAFNNGSGSGDGIDKPVVPEQPDEPGEIEPIPE

SEQ ID NO:11

SLAAVAADAPVAGTDITNQLTNVTVGIDSGTTVYPHQAGYVKLNYGFSVPNSAVKGDTFKITVPKELNLNGVT
STAKVPPIMAGDQVLANGVIDSDGNVIYTFTDYVNTKDDVKATLTMSAAIDPENVKKTGNVTLATGIGSTTAN
KTVLVDYEKYGKFYNLSIKGTIDQIDKTNNTYRQTIYVNPSGDNVIAPVLTGNLKPNTDSNALIDQQNTSIKV
YKVDNAADLSESYFVNPENFEDVTNSVNITFPNPNQYKVEFNTPDDQITTPYIVVVNGHIDPNSKGDLALRST
LYGYNSNIIWRSMSWDNEVAFNNGSGSGDGIDKPVVPEQPDEPGEIEPIPE

SEQ ID NO:12

GTDITNQLTNVTVGIDSGTTVYPHQAGYVKLNYGFSVPNSAVKGDTFKITVPKELNLNGVTSTAKVPPIMAG
DQVLANGVIDSDGNVIYTFTDYVNTKDDVKATLTMSAAIDPENVKKTGNVTLATGIGSTTANKTVLVDYEKYG
KFYNLSIKGTIDQIDKTNNTYRQTIYVNPSGDNVIAPVLTGNLKPNTDSNALIDQQNTSIKVYKVDNAADLSE
SYFVNPENFEDVTNSVNITFPNPNQYKVEFNTPDDQITTPYIVVVNGHIDPNSKGDLALRSTLYGYNSNIIWR
SMSWDNEVAFNNGSGSGDGIDKPVVPEQPDEPGEIEPIPE

SEQ ID NO:13

MKTRIVSSVTTTLLLGSILMNPVANAADSDINIKTGTTDIGSNTTVKTGDLVTYDKENGMRKKVFY
SFIDDKNHNKKLLVIRTKGTIAGQYRVYSEEGANKSGLAWPSAFKVQLQLPDNEVAQISDYYPRNS
IDTKEYMSTLTYGFNGNVTGDDTGKIGGLIGANVSIGHTLKYVQPDFKTILESPTDKKVGWKVIFN
NMVNQNWGPYDRDSWNPVYGNQLFMKTRNGSMKAADNFLDPNKASSLLSSGFSPDFATVITMDRKA
SKQQTNIDVIYERVRDDYQLHWTSTNWKGTNTKDKWIDRSSERYKIDWEKEEMTN

SEQ ID NO:14
MADSDINIKTGTTDIGSNTTVKTGDLVTYDKENGMRKKVFYSFIDDKNHNKKLLVIRTKGTIAGQY
RVYSEEGANKSGLAWPSAFKVQLQLPDNEVAQISDYYPRNSIDTKEYMSTLTYGFNGNVTGDDTGK
IGGLIGANVSIGHTLKYVQPDFKTILESPTDKKVGWKVIFNNMVNQNWGPYDRDSWNPVYGNQLFM
KTRNGSMKAADNFLDPNKASSLLSSGFSPDFATVITMDRKASKQQTNIDVIYERVRDDYQLHWTST
NWKGTNTKDKWIDRSSERYKIDWEKEEMTN

SEQ ID NO:15

MASLAAVAADAPVAGTDITNQLTNVTVGIDSGTTVYPHQAGYVKLNYGFSVPNSAVKGDTFKITVPKELNLNG
VTSTAKVPPIMAGDQVLANGVIDSDGNVIYTFTDYVNTKDDVKATLTMPAYIDPENVKKTGNVTLATGIGSTT
ANKTVLVDYEKYGKFYNLSIKGTIDQIDKTNNTYRQTIYVNPSGDNVIAPVLTGNLKPNTDSNALIDQQNTSI
KVYKVDNAADLSESYFVNPENFEDVTNSVNITFPNPNQYKVEFNTPDDQITTPYIVVVNGHIDPNSKGDLALR
STLYGYNSNIIWRSMSWDNEVAFNNGSGSGDGIDKPVVPEQPDEPGEIEPIPE

SEQ ID NO:16

MAGTDITNQLTNVTVGIDSGTTVYPHQAGYVKLNYGFSVPNSAVKGDTFKITVPKELNLNGVTSTAKVPPIMA
GDQVLANGVIDSDGNVIYTFTDYVNTKDDVKATLTMPAYIDPENVKKTGNVTLATGIGSTTANKTVLVDYEKY
GKFYNLSIKGTIDQIDKTNNTYRQTIYVNPSGDNVIAPVLTGNLKPNTDSNALIDQQNTSIKVYKVDNAADLS
ESYFVNPENFEDVTNSVNITFPNPNQYKVEFNTPDDQITTPYIVVVNGHIDPNSKGDLALRSTLYGYNSNIIW
RSMSWDNEVAFNNGSGSGDGIDKPVVPEQPDEPGEIEPIPE

SEQ ID NO:17

MASLAAVAADAPVAGTDITNQLTNVTVGIDSGTTVYPHQAGYVKLNYGFSVPNSAVKGDTFKITVPKELNLNG
VTSTAKVPPIMAGDQVLANGVIDSDGNVIYTFTDYVNTKDDVKATLTMSAAIDPENVKKTGNVTLATGIGSTT
ANKTVLVDYEKYGKFYNLSIKGTIDQIDKTNNTYRQTIYVNPSGDNVIAPVLTGNLKPNTDSNALIDQQNTSI
KVYKVDNAADLSESYFVNPENFEDVTNSVNITFPNPNQYKVEFNTPDDQITTPYIVVVNGHIDPNSKGDLALR
STLYGYNSNIIWRSMSWDNEVAFNNGSGSGDGIDKPVVPEQPDEPGEIEPIPE

SEQ ID NO:18

MAGTDITNQLTNVTVGIDSGTTVYPHQAGYVKLNYGFSVPNSAVKGDTFKITVPKELNLNGVTSTAKVPPIMA
GDQVLANGVIDSDGNVIYTFTDYVNTKDDVKATLTMSAAIDPENVKKTGNVTLATGIGSTTANKTVLVDYEKY
GKFYNLSIKGTIDQIDKTNNTYRQTIYVNPSGDNVIAPVLTGNLKPNTDSNALIDQQNTSIKVYKVDNAADLS
ESYFVNPENFEDVTNSVNITFPNPNQYKVEFNTPDDQITTPYIVVVNGHIDPNSKGDLALRSTLYGYNSNIIW
RSMSWDNEVAFNNGSGSGDGIDKPVVPEQPDEPGEIEPIPE

### Example 2 Preparation of vaccine components

A four component staphylococcal vaccine was prepared which contained *S. aureus* Type 5 capsular polysaccharide conjugated to a tetanus toxoid carrier protein, *S. aureus* Type 8 capsular polysaccharide conjugated to a tetanus toxoid carrier protein, a fragment of ClfA containing the N2 and N3 domains and point mutations at residues 336 and 338 in which P336 is changed to serine and Y338 is changed to alanine, and alpha toxoid which is detoxified by a point mutation at residue 35 with H35 being changed to arginine. The capsular polysaccharides were conjugated to tetanus toxoid using CDAP as the coupling agent. This conjugation process is described in WO 07/113222.

Four formulations of the staphylococcal vaccine were made:
**5/10 contained:** 5µg saccharide dose of Type 5 - tetanus toxoid conjugate, 5µg saccharide dose of Type 8 - tetanus toxoid conjugate, 10µg of alpha toxoid and 10µg of the ClfA truncate described above.
**10/30 contained:** 10µg saccharide dose of Type 5 - tetanus toxoid conjugate, 10µg saccharide dose of Type 8 - tetanus toxoid conjugate, 30µg of alpha toxoid and 30µg of the ClfA truncate described above.
**5/10AS contained:** 5µg saccharide dose of Type 5 - tetanus toxoid conjugate, 5µg saccharide dose of Type 8 - tetanus toxoid conjugate, 10µg of alpha toxoid and 10µg of the ClfA truncate described above, adjuvanted with an oil in water elusion containing squalene, alpha-tocopherol and polyoxyethylene sorbitan monooleate.
**10/30AS contained:** 10µg saccharide dose of Type 5 - tetanus toxoid conjugate, 10µg saccharide dose of Type 8 - tetanus toxoid conjugate, 30µg of alpha toxoid and 30µg of the ClfA truncate described above, adjuvanted with an oil in water elusion containing squalene, alpha-tocopherol and polyoxyethylene sorbitan monooleate.

### Example 3 Clinical trial results using the 4 component staphylococcal vaccine

A phase I clinical trial was carried out using a total of 88 healthy adults from 18 to 40 years old. The control group contained 30 subjects who were inoculated with saline. The remaining subjects were divided into four arms with 15/14 subjects being immunised with each of the formulations described in example 2 (5/10, 5/10AS, 10/30 and 10/30AS). Vaccine doses were given at the start of the trial and after one month and at six months. Blood samples for humoral analysis were taken at day 0, 7, 14 and 30 after each dose and at day 360 and 540.

Details of the subjects are provided below.

| Group | N | Mean Age | % female |
|---|---|---|---|
| 5/10 | 15 | 31.1 | 73.3 |
| 5/10AS | 15 | 31.9 | 33.3 |
| 10/30 | 14 | 30.9 | 42.9 |
| 10/30AS | 14 | 30.6 | 50 |
| Saline | 30 | 30.1 | 50 |

### Reactogenicity and Safety

The 4 component staphylococcal vaccine was generally safe and well tolerated. After the fist and second doses no serious adverse events and no potential immune mediated disorders were observed. The percentage of subjects reporting pain, redness and swelling after dose 1 and dose 2 is shown in Figures 1-3. Pain was experienced at the injection site in 78.6-100% of subjects in the vaccine groups compared to 3-4% in the control group (see Figure 1). However, only one case was graded 3. Results for the incidence of redness and swelling are shown in Figures 2 and 3. For both parameters, there was a trend for a higher incidence of redness/swelling following administration of the second dose compared to after a single dose for the 10/30 arm of the study.

### Immunogenicity

Blood samples taken from subjects on day 0 and 7, 14 and 30 days following the first second and third immunisations were tested by Luminex or ELISA to establish the level of IgG produced against each antigen of the four component staphylococcal vaccine.

Results for immunogenicity are shown in Figures 4-8 and in the Tables 1-5 below.

Prevaccination, there was 83.3-100% seropositivity for all assays. Despite considerable levels of background immunity, the 4 component vaccine was able to elicit a robust immune response against all 4 components.

Figures 4-7 show that for CPS5, CPS8, alpha toxoid and ClfA, the first immunisation produced the largest increase in immunogenicity with strong increases of GMC being apparent at day 14 and 30. The second immunisation on day 30 did not produce a further increase in immunogenicity and GMC levels remain at a similar level between days 30 and 60. Figure 8 shows that the third immunisation after 6 months did not provoke a further increase in GMC with GMC levels remaining approximately the same for the four components between day 30 and day 540. A single immunisation is therefore an efficient way of producing a maximal immune response.

The immunogenicity results for the 10/30 dosage appear to be stronger than for the 5/10 dosage with an approximately 1-5-2 fold increase of GMC for CPS5, CPS8 and alpha toxoid. In the case of ClfA the increase in GMC was about 3.8 fold at the higher dose. The addition of oil in water emulsion adjuvant did not increase the immunogenicity of the 4 component vaccine as demonstrated by a comparison of antibody response elicited by the 5/10 and 5/10AS arms and the 10/30 and 10/30AS arms.

**Table 1 Seropositivity rates and GMCs for Staph aureus.CPS 5 Ab.IgG antibodies (ATP cohort for immunogenicity)**

| | | | | **≥ 23.6 LU/ml** | | | | **GMC** | | |
|---|---|---|---|---|---|---|---|---|---|---|
| | | | | | | **95% Cl** | | | **95% Cl** | |
| **Antibody** | **Group** | **Timing** | **N** | **n** | **%** | **LL** | **UL** | **value** | **LL** | **UL** |
| Staph aureus.CPS 5 Ab.IgG | 5/10 | PRE | 15 | 13 | 86.7 | 59.5 | 98.3 | 104.00 | 51.24 | 211.07 |
| | | PI(D7) | 15 | 14 | 93.3 | 68.1 | 99.8 | 702.89 | 316.09 | 1562.98 |
| | | PI(D14) | 11 | 11 | 100 | 71.5 | 100 | 2393.81 | 1164.68 | 4920.09 |
| | | PI(D30) | 14 | 14 | 100 | 76.8 | 100 | 3515.50 | 1690.01 | 7312.81 |
| | | PII(D37) | 9 | 9 | 100 | 66.4 | 100 | 3970.84 | 1570.67 | 10038.80 |
| | | PII(D44) | 9 | 9 | 100 | 66.4 | 100 | 3485.16 | 1456.13 | 8341.54 |
| | | PII(D60) | 9 | 9 | 100 | 66.4 | 100 | 3648.17 | 1414.59 | 9408.46 |
| | 5/10AS | PRE | 15 | 14 | 93.3 | 68.1 | 99.8 | 175.35 | 77.12 | 398.69 |
| | | PI(D7) | 15 | 15 | 100 | 78.2 | 100 | 1745.15 | 1016.89 | 2994.97 |
| | | PI(D14) | 15 | 15 | 100 | 78.2 | 100 | 5447.98 | 3150.01 | 9422.35 |
| | | PI(D30) | 15 | 15 | 100 | 78.2 | 100 | 4962.11 | 2766.72 | 8899.55 |
| | | PII(D37) | 12 | 12 | 100 | 73.5 | 100 | 3831.22 | 2234.21 | 6569.79 |
| | | PII(D44) | 12 | 12 | 100 | 73.5 | 100 | 4262.74 | 2373.12 | 7656.98 |
| | | PII(D60) | 12 | 12 | 100 | 73.5 | 100 | 3920.80 | 2316.00 | 6637.61 |
| | 10/30 | PRE | 14 | 14 | 100 | 76.8 | 100 | 114.74 | 60.89 | 216.23 |
| | | PI(D7) | 6 | 6 | 100 | 54.1 | 100 | 1231.04 | 342.92 | 4419.30 |
| | | PI(D14) | 11 | 11 | 100 | 71.5 | 100 | 6684.54 | 4060.86 | 11003.35 |
| | | PI(D30) | 14 | 14 | 100 | 76.8 | 100 | 5023.61 | 2922.27 | 8636.00 |
| | | PII(D37) | 12 | 12 | 100 | 73.5 | 100 | 6228.11 | 3904.47 | 9934.61 |
| | | PII(D44) | 12 | 12 | 100 | 73.5 | 100 | 6625.99 | 4026.07 | 10904.85 |
| | | PII(D60) | 12 | 12 | 100 | 73.5 | 100 | 5749.41 | 3442.63 | 9601.86 |
| | 10/30AS | PRE | 14 | 13 | 92.9 | 66.1 | 99.8 | 114.02 | 48.87 | 266.02 |
| | | PI(D7) | 6 | 6 | 100 | 54.1 | 100 | 4088.58 | 2215.34 | 7545.81 |
| | | PI(D14) | 11 | 11 | 100 | 71.5 | 100 | 7598.72 | 4120.90 | 14011.61 |
| | | PI(D30) | 14 | 14 | 100 | 76.8 | 100 | 5569.08 | 2994.06 | 10358.73 |
| | | PII(D37) | 13 | 13 | 100 | 75.3 | 100 | 5930.99 | 3425.26 | 10269.76 |
| | | PII(D44) | 13 | 13 | 100 | 75.3 | 100 | 6588.83 | 3645.17 | 11909.64 |
| | | PII(D60) | 13 | 13 | 100 | 75.3 | 100 | 6582.67 | 3229.11 | 13419.03 |
| | SALINE | PRE | 30 | 25 | 83.3 | 65.3 | 94.4 | 79.19 | 46.96 | 133.54 |
| | | PI(D7) | 29 | 23 | 79.3 | 60.3 | 92.0 | 80.62 | 45.45 | 143.00 |
| | | PI(D14) | 29 | 23 | 79.3 | 60.3 | 92.0 | 80.57 | 46.22 | 140.43 |
| | | PI(D30) | 30 | 24 | 80.0 | 61.4 | 92.3 | 85.65 | 49.13 | 149.31 |
| | | PII(D37) | 24 | 20 | 83.3 | 62.6 | 95.3 | 65.60 | 38.22 | 112.60 |
| | | PII(D44) | 23 | 19 | 82.6 | 61.2 | 95.0 | 62.84 | 35.17 | 112.30 |
| | | PII(D60) | 24 | 18 | 75.0 | 53.3 | 90.2 | 60.24 | 33.89 | 107.06 |

| | | | | | | | | | | |
|---|---|---|---|---|---|---|---|---|---|---|
| 5/10 = 5µg CPS5-TT, 5µg CPS8-TT, 10µg ClfA, 10µg α-toxoid 5/10AS = 5µg CPS5-TT, 5µg CPS8-TT, 10µg ClfA, 10µg α-toxoid adjuvanted with AS03B 10/30 = 10µg CPS5-TT, 10µg CPS8-TT, 30µg ClfA, 30µg α-toxoid 10/30AS = 10µg CPS5-TT, 10µg CPS8-TT, 30µg ClfA, 30µg α-toxoid adjuvanted with AS03B SALINE = pooling of SALINE1 and SALINE2 GMC = geometric mean antibody concentration calculated on all subjects N = number of subjects with available results n/% = number/percentage of subjects with concentration within the specified range 95% Cl = 95% confidence interval; LL = Lower Limit, UL = Upper Limit PRE = pre dose 1 PI(D7) = 7 days post dose 1 PI(D14) = 14 days post dose 1 PI(D30) = 30 days post dose 1 (blood sample taken at Visits 5 or 6) PII(D37) = 7 days post dose 2 PII(D44) = 14 days post dose 2 PII(D60) = 30 days post dose 2 | | | | | | | | | | |

**Table 2 Seropositivity rates and GMCs for Staph aureus.CPS 8 Ab.IgG antibodies (ATP cohort for immunogenicity)**

| | | | | **≥ 26.5 LU/ml** | | | | **GMC** | | |
|---|---|---|---|---|---|---|---|---|---|---|
| | | | | | | **95% Cl** | | | **95% Cl** | |
| **Antibody** | **Group** | **Timing** | **N** | **n** | **%** | **LL** | **UL** | **value** | **LL** | **UL** |
| Staph aureus.CPS 8 Ab.IgG | 5/10 | PRE | 15 | 14 | 93.3 | 68.1 | 99.8 | 377.47 | 176.95 | 805.24 |
| | | PI(D7) | 15 | 15 | 100 | 78.2 | 100 | 1101.09 | 460.23 | 2634.33 |
| | | PI(D14) | 14 | 14 | 100 | 76.8 | 100 | 3151.09 | 1460.34 | 6799.36 |
| | | PI(D30) | 15 | 15 | 100 | 78.2 | 100 | 3169.43 | 1471.27 | 6827.61 |
| | | PII(D37) | 10 | 10 | 100 | 69.2 | 100 | 4382.17 | 2147.01 | 8944.26 |
| | | PII(D44) | 10 | 10 | 100 | 69.2 | 100 | 3776.90 | 2035.45 | 7008.27 |
| | | PII(D60) | 10 | 10 | 100 | 69.2 | 100 | 4120.46 | 2329.69 | 7287.77 |
| | 5/10AS | PRE | 15 | 15 | 100 | 78.2 | 100 | 533.66 | 270.37 | 1053.36 |
| | | PI(D7) | 15 | 15 | 100 | 78.2 | 100 | 2220.14 | 1489.78 | 3308.56 |
| | | PI(D14) | 13 | 13 | 100 | 75.3 | 100 | 4831.66 | 3164.57 | 7376.97 |
| | | PI(D30) | 13 | 13 | 100 | 75.3 | 100 | 4328.02 | 2494.84 | 7508.20 |
| | | PII(D37) | 11 | 11 | 100 | 71.5 | 100 | 3722.46 | 2425.65 | 5712.58 |
| | | PII(D44) | 11 | 11 | 100 | 71.5 | 100 | 3973.72 | 2364.01 | 6679.54 |
| | | PII(D60) | 11 | 11 | 100 | 71.5 | 100 | 3573.72 | 2256.18 | 5660.67 |
| | 10/30 | PRE | 12 | 12 | 100 | 73.5 | 100 | 446.48 | 189.79 | 1050.34 |
| | | PI(D7) | 12 | 12 | 100 | 73.5 | 100 | 2830.32 | 1540.49 | 5200.12 |
| | | PI(D14) | 14 | 14 | 100 | 76.8 | 100 | 9038.91 | 5796.13 | 14095.93 |
| | | PI(D30) | 13 | 13 | 100 | 75.3 | 100 | 7980.64 | 5159.87 | 12343.44 |
| | | PII(D37) | 12 | 12 | 100 | 73.5 | 100 | 7205.23 | 4676.27 | 11101.87 |
| | | PII(D44) | 12 | 12 | 100 | 73.5 | 100 | 7549.64 | 4717.98 | 12080.83 |
| | | PII(D60) | 11 | 11 | 100 | 71.5 | 100 | 6728.09 | 4425.54 | 10228.61 |
| | 10/30AS | PRE | 14 | 12 | 85.7 | 57.2 | 98.2 | 207.57 | 81.34 | 529.65 |
| | | PI(D7) | 11 | 11 | 100 | 71.5 | 100 | 2049.03 | 769.73 | 5454.51 |
| | | PI(D14) | 12 | 12 | 100 | 73.5 | 100 | 6569.22 | 3215.77 | 13419.68 |
| | | PI(D30) | 13 | 13 | 100 | 75.3 | 100 | 5307.09 | 2468.17 | 11411.40 |
| | | PII(D37) | 13 | 13 | 100 | 75.3 | 100 | 5984.18 | 3461.54 | 10345.20 |
| | | PII(D44) | 12 | 12 | 100 | 73.5 | 100 | 6549.44 | 3543.91 | 12103.91 |
| | | PII(D60) | 12 | 12 | 100 | 73.5 | 100 | 6665.14 | 3418.24 | 12996.20 |
| | SALINE | PRE | 28 | 26 | 92.9 | 76.5 | 99.1 | 335.46 | 184.17 | 611.03 |
| | | PI(D7) | 27 | 25 | 92.6 | 75.7 | 99.1 | 340.15 | 182.56 | 633.77 |
| | | PI(D14) | 28 | 26 | 92.9 | 76.5 | 99.1 | 355.41 | 195.74 | 645.30 |
| | | PI(D30) | 30 | 29 | 96.7 | 82.8 | 99.9 | 362.15 | 210.58 | 622.79 |
| | | PII(D37) | 24 | 22 | 91.7 | 73.0 | 99.0 | 361.33 | 182.65 | 714.82 |
| | | PII(D44) | 23 | 22 | 95.7 | 78.1 | 99.9 | 418.45 | 216.00 | 810.66 |
| | | PII(D60) | 24 | 23 | 95.8 | 78.9 | 99.9 | 368.24 | 189.56 | 715.34 |

| | | | | | | | | | | |
|---|---|---|---|---|---|---|---|---|---|---|
| 5/10 = 5µg CPS5-TT, 5µg CPS8-TT, 10µg ClfA, 10µg α-toxoid 5/10AS = 5µg CPS5-TT, 5µg CPS8-TT, 10µg ClfA, 10µg α-toxoid adjuvanted with AS03B 10/30 = 10µg CPS5-TT, 10µg CPS8-TT, 30µg ClfA, 30µg α-toxoid 10/30AS = 10µg CPS5-TT, 10µg CPS8-TT, 30µg ClfA, 30µg α-toxoid adjuvanted with AS03B SALINE = pooling of SALINE1 and SALINE2 GMC = geometric mean antibody concentration calculated on all subjects N = number of subjects with available results n/% = number/percentage of subjects with concentration within the specified range 95% Cl = 95% confidence interval; LL = Lower Limit, UL = Upper Limit PRE = pre dose 1 PI(D7) = 7 days post dose 1 PI(D14) = 14 days post dose 1 PI(D30) = 30 days post dose 1 (blood sample taken at Visits 5 or 6) PII(D37) = 7 days post dose 2 PII(D44) = 14 days post dose 2 PII(D60) = 30 days post dose 2 | | | | | | | | | | |

**Table 3 Seropositivity rates and GMCs for Staph aureus alpha-toxin Ab.IgG antibodies (ATP cohort for immunogenicity)**

| | | | | **≥ 22.5 LU/ml** | | | | **GMC** | | |
|---|---|---|---|---|---|---|---|---|---|---|
| | | | | | | **95% CI** | | | **95% CI** | |
| **Antibody** | **Group** | **Timing** | **N** | **n** | **%** | **LL** | **UL** | **value** | **LL** | **UL** |
| Staph aureus alpha-toxin Ab.IgG | 5/10 | PRE | 15 | 15 | 100 | 78.2 | 100 | 181.59 | 112.62 | 292.80 |
| | | PI(D7) | 15 | 15 | 100 | 78.2 | 100 | 508.56 | 342.65 | 754.79 |
| | | PI(D14) | 14 | 14 | 100 | 76.8 | 100 | 924.97 | 617.24 | 1386.12 |
| | | PI(D30) | 15 | 15 | 100 | 78.2 | 100 | 946.86 | 654.84 | 1369.11 |
| | | PII(D37) | 10 | 10 | 100 | 69.2 | 100 | 991.85 | 565.44 | 1739.82 |
| | | PII(D44) | 10 | 10 | 100 | 69.2 | 100 | 885.68 | 595.57 | 1317.12 |
| | | PII(D60) | 10 | 10 | 100 | 69.2 | 100 | 960.49 | 615.68 | 1498.42 |
| | 5/10AS | PRE | 15 | 15 | 100 | 78.2 | 100 | 212.93 | 142.13 | 318.99 |
| | | PI(D7) | 15 | 15 | 100 | 78.2 | 100 | 639.16 | 441.46 | 925.40 |
| | | PI(D14) | 15 | 15 | 100 | 78.2 | 100 | 910.41 | 586.44 | 1413.34 |
| | | PI(D30) | 15 | 15 | 100 | 78.2 | 100 | 842.98 | 594.48 | 1195.38 |
| | | PII(D37) | 12 | 12 | 100 | 73.5 | 100 | 974.08 | 644.36 | 1472.52 |
| | | PII(D44) | 12 | 12 | 100 | 73.5 | 100 | 1134.60 | 745.18 | 1727.54 |
| | | PII(D60) | 12 | 12 | 100 | 73.5 | 100 | 1048.51 | 693.13 | 1586.12 |
| | 10/30 | PRE | 11 | 11 | 100 | 71.5 | 100 | 339.09 | 200.20 | 574.32 |
| | | PI(D7) | 13 | 13 | 100 | 75.3 | 100 | 919.07 | 543.30 | 1554.74 |
| | | PI(D14) | 13 | 13 | 100 | 75.3 | 100 | 2534.87 | 1728.09 | 3718.31 |
| | | PI(D30) | 14 | 14 | 100 | 76.8 | 100 | 1913.52 | 1224.06 | 2991.33 |
| | | PII(D37) | 12 | 12 | 100 | 73.5 | 100 | 1804.43 | 1163.54 | 2798.33 |
| | | PII(D44) | 12 | 12 | 100 | 73.5 | 100 | 1988.02 | 1326.61 | 2979.21 |
| | | PII(D60) | 12 | 12 | 100 | 73.5 | 100 | 1947.83 | 1295.34 | 2929.01 |
| | 10/30AS | PRE | 13 | 13 | 100 | 75.3 | 100 | 232.25 | 132.26 | 407.85 |
| | | PI(D7) | 12 | 12 | 100 | 73.5 | 100 | 920.78 | 539.84 | 1570.55 |
| | | PI(D14) | 13 | 13 | 100 | 75.3 | 100 | 1569.68 | 980.44 | 2513.05 |
| | | PI(D30) | 14 | 14 | 100 | 76.8 | 100 | 1251.47 | 800.34 | 1956.89 |
| | | PII(D37) | 13 | 13 | 100 | 75.3 | 100 | 1508.59 | 1021.42 | 2228.12 |
| | | PII(D44) | 13 | 13 | 100 | 75.3 | 100 | 1779.93 | 1287.31 | 2461.06 |
| | | PII(D60) | 13 | 13 | 100 | 75.3 | 100 | 1936.73 | 1356.02 | 2766.13 |
| | SALINE | PRE | 30 | 28 | 93.3 | 77.9 | 99.2 | 284.13 | 181.05 | 445.91 |
| | | PI(D7) | 27 | 26 | 96.3 | 81.0 | 99.9 | 306.37 | 186.96 | 502.02 |
| | | PI(D14) | 28 | 27 | 96.4 | 81.7 | 99.9 | 308.14 | 193.80 | 489.93 |
| | | PI(D30) | 30 | 29 | 96.7 | 82.8 | 99.9 | 285.96 | 187.27 | 436.64 |
| | | PII(D37) | 24 | 23 | 95.8 | 78.9 | 99.9 | 268.62 | 160.19 | 450.46 |
| | | PII(D44) | 23 | 22 | 95.7 | 78.1 | 99.9 | 281.86 | 173.60 | 457.65 |
| | | PII(D60) | 24 | 22 | 91.7 | 73.0 | 99.0 | 260.11 | 153.51 | 440.75 |

| | | | | | | | | | | |
|---|---|---|---|---|---|---|---|---|---|---|
| 5/10 = 5µg CPS5-TT, 5µg CPS8-TT, 10µg ClfA, 10µg α-toxoid 5/10AS = 5µg CPS5-TT, 5µg CPS8-TT, 10µg ClfA, 10µg α-toxoid adjuvanted with AS03B 10/30 = 10µg CPS5-TT, 10µg CPS8-TT, 30µg ClfA, 30µg α-toxoid 10/30AS = 10µg CPS5-TT, 10µg CPS8-TT, 30µg ClfA, 30µg α-toxoid adjuvanted with AS03B SALINE = pooling of SALINE1 and SALINE2 GMC = geometric mean antibody concentration calculated on all subjects N = number of subjects with available results n/% = number/percentage of subjects with concentration within the specified range 95% Cl = 95% confidence interval; LL = Lower Limit, UL = Upper Limit PRE = pre dose 1 PI(D7) = 7 days post dose 1 PI(D14) = 14 days post dose 1 PI(D30) = 30 days post dose 1 (blood sample taken at Visits 5 or 6) PII(D37) = 7 days post dose 2 PII(D44) = 14 days post dose 2 PII(D60) = 30 days post dose 2 | | | | | | | | | | |

**Table 4 Seropositivity rates and GMCs for Staph aureus ClfA Ab.IgG antibodies (ATP cohort for immunogenicity)**

| | | | | **≥ 6 ELU/ml** | | | | **GMC** | | |
|---|---|---|---|---|---|---|---|---|---|---|
| | | | | | | **95% CI** | | | **95% CI** | |
| **Antibody** | **Group** | **Timing** | **N** | **n** | **%** | **LL** | **UL** | **value** | **LL** | **UL** |
| Staph aureus.ClfA Ab.IgG | 5/10 | PRE | 15 | 15 | 100 | 78.2 | 100 | 58.10 | 31.62 | 106.74 |
| | | PI(D7) | 15 | 15 | 100 | 78.2 | 100 | 364.64 | 150.30 | 884.67 |
| | | PI(D14) | 14 | 14 | 100 | 76.8 | 100 | 2830.51 | 958.28 | 8360.54 |
| | | PI(D30) | 15 | 15 | 100 | 78.2 | 100 | 3785.71 | 1599.23 | 8961.54 |
| | | PII(D37) | 10 | 10 | 100 | 69.2 | 100 | 4495.84 | 2297.39 | 8798.06 |
| | | PII(D44) | 10 | 10 | 100 | 69.2 | 100 | 5472.85 | 3165.82 | 9461.09 |
| | | PII(D60) | 10 | 10 | 100 | 69.2 | 100 | 4889.94 | 2758.53 | 8668.20 |
| | 5/10AS | PRE | 15 | 15 | 100 | 78.2 | 100 | 128.80 | 81.19 | 204.34 |
| | | PI(D7) | 15 | 15 | 100 | 78.2 | 100 | 1271.87 | 629.74 | 2568.79 |
| | | PI(D14) | 15 | 15 | 100 | 78.2 | 100 | 5967.39 | 3036.36 | 11727.76 |
| | | PI(D30) | 15 | 15 | 100 | 78.2 | 100 | 6580.65 | 3474.92 | 12462.12 |
| | | PII(D37) | 12 | 12 | 100 | 73.5 | 100 | 9654.46 | 5153.40 | 18086.81 |
| | | PII(D44) | 12 | 12 | 100 | 73.5 | 100 | 9852.33 | 5477.46 | 17721.43 |
| | | PII(D60) | 12 | 12 | 100 | 73.5 | 100 | 9875.62 | 5738.09 | 16996.56 |
| | 10/30 | PRE | 14 | 14 | 100 | 76.8 | 100 | 101.38 | 70.70 | 145.39 |
| | | PI(D7) | 14 | 14 | 100 | 76.8 | 100 | 861.08 | 471.92 | 1571.15 |
| | | PI(D14) | 14 | 14 | 100 | 76.8 | 100 | 6627.23 | 3291.32 | 13344.28 |
| | | PI(D30) | 14 | 14 | 100 | 76.8 | 100 | 8068.07 | 4029.42 | 16154.63 |
| | | PII(D37) | 12 | 12 | 100 | 73.5 | 100 | 8465.30 | 4124.58 | 17374.21 |
| | | PII(D44) | 12 | 12 | 100 | 73.5 | 100 | 9130.37 | 4769.02 | 17480.23 |
| | | PII(D60) | 12 | 12 | 100 | 73.5 | 100 | 9840.83 | 5320.61 | 18201.28 |
| | 10/30AS | PRE | 14 | 14 | 100 | 76.8 | 100 | 86.57 | 56.65 | 132.29 |
| | | PI(D7) | 14 | 14 | 100 | 76.8 | 100 | 1097.71 | 550.91 | 2187.24 |
| | | PI(D14) | 14 | 14 | 100 | 76.8 | 100 | 6472.06 | 3731.51 | 11225.35 |
| | | PI(D30) | 14 | 14 | 100 | 76.8 | 100 | 6376.38 | 3505.45 | 11598.55 |
| | | PII(D37) | 13 | 13 | 100 | 75.3 | 100 | 6673.11 | 3836.01 | 11608.50 |
| | | PII(D44) | 13 | 13 | 100 | 75.3 | 100 | 7724.57 | 4739.23 | 12590.44 |
| | | PII(D60) | 13 | 13 | 100 | 75.3 | 100 | 8067.05 | 4906.74 | 13262.83 |
| | SALINE | PRE | 30 | 28 | 93.3 | 77.9 | 99.2 | 80.71 | 46.62 | 139.75 |
| | | PI(D7) | 30 | 28 | 93.3 | 77.9 | 99.2 | 83.79 | 48.36 | 145.18 |
| | | PI(D14) | 30 | 29 | 96.7 | 82.8 | 99.9 | 87.81 | 51.46 | 149.82 |
| | | PI(D30) | 30 | 28 | 93.3 | 77.9 | 99.2 | 91.86 | 52.48 | 160.77 |
| | | PII(D37) | 24 | 22 | 91.7 | 73.0 | 99.0 | 78.61 | 40.78 | 151.52 |
| | | PII(D44) | 23 | 21 | 91.3 | 72.0 | 98.9 | 83.41 | 43.67 | 159.32 |
| | | PII(D60) | 24 | 22 | 91.7 | 73.0 | 99.0 | 81.29 | 42.24 | 156.47 |

| | | | | | | | | | | |
|---|---|---|---|---|---|---|---|---|---|---|
| 5/10 = 5µg CPS5-TT, 5µg CPS8-TT, 10µg ClfA, 10µg α-toxoid 5/10AS = 5µg CPS5-TT, 5µg CPS8-TT, 10µg ClfA, 10µg α-toxoid adjuvanted with AS03B 10/30 = 10µg CPS5-TT, 10µg CPS8-TT, 30µg ClfA, 30µg α-toxoid 10/30AS = 10µg CPS5-TT, 10µg CPS8-TT, 30µg ClfA, 30µg α-toxoid adjuvanted with AS03B SALINE = pooling of SALINE1 and SALINE2 GMC = geometric mean antibody concentration calculated on all subjects N = number of subjects with available results n/% = number/percentage of subjects with concentration within the specified range 95% Cl = 95% confidence interval; LL = Lower Limit, UL = Upper Limit PRE = pre dose 1 PI(D7) = 7 days post dose 1 PI(D14) = 14 days post dose 1 PI(D30) = 30 days post dose 1 (blood sample taken at Visits 5 or 6) PII(D37) = 7 days post dose 2 PII(D44) = 14 days post dose 2 PII(D60) = 30 days post dose 2 | | | | | | | | | | |

**Table 5 Seropositivity rates and GMCs for C tetani.Tox Ab.IgG antibodies (ATP cohort for immunogenicity)**

| | | | | **≥ 0.1 IU/ml** | | | | **GMC** | | |
|---|---|---|---|---|---|---|---|---|---|---|
| | | | | | | **95% CI** | | | **95% CI** | |
| **Antibody** | **Group** | **Timinq** | **N** | **n** | **%** | **LL** | **UL** | **value** | **LL** | **UL** |
| C tetani.Tox Ab.IgG | 5/10 | PRE | 15 | 13 | 86.7 | 59.5 | 98.3 | 1.071 | 0.366 | 3.139 |
| | | PI(D7) | 15 | 15 | 100 | 78.2 | 100 | 5.125 | 2.687 | 9.777 |
| | | PI(D14) | 14 | 14 | 100 | 76.8 | 100 | 11.070 | 7.188 | 17.047 |
| | | PI(D30) | 15 | 15 | 100 | 78.2 | 100 | 8.324 | 5.200 | 13.325 |
| | | PII(D37) | 10 | 10 | 100 | 69.2 | 100 | 7.516 | 3.585 | 15.756 |
| | | PII(D44) | 10 | 10 | 100 | 69.2 | 100 | 6.909 | 3.469 | 13.757 |
| | | PII(D60) | 10 | 10 | 100 | 69.2 | 100 | 5.582 | 2.473 | 12.601 |
| | 5/10AS | PRE | 15 | 14 | 93.3 | 68.1 | 99.8 | 2.010 | 0.879 | 4.600 |
| | | PI(D7) | 15 | 15 | 100 | 78.2 | 100 | 7.096 | 4.799 | 10.494 |
| | | PI(D14) | 15 | 15 | 100 | 78.2 | 100 | 10.545 | 7.732 | 14.382 |
| | | PI(D30) | 15 | 15 | 100 | 78.2 | 100 | 9.249 | 6.845 | 12.497 |
| | | PII(D37) | 12 | 12 | 100 | 73.5 | 100 | 8.530 | 6.265 | 11.615 |
| | | PII(D44) | 12 | 12 | 100 | 73.5 | 100 | 8.906 | 5.604 | 14.154 |
| | | PII(D60) | 12 | 12 | 100 | 73.5 | 100 | 8.600 | 5.470 | 13.521 |
| | 10/30 | PRE | 14 | 13 | 92.9 | 66.1 | 99.8 | 3.264 | 1.225 | 8.698 |
| | | PI(D7) | 14 | 14 | 100 | 76.8 | 100 | 16.200 | 10.728 | 24.463 |
| | | PI(D14) | 14 | 14 | 100 | 76.8 | 100 | 22.716 | 14.191 | 36.364 |
| | | PI(D30) | 14 | 14 | 100 | 76.8 | 100 | 16.495 | 10.461 | 26.010 |
| | | PII(D37) | 12 | 12 | 100 | 73.5 | 100 | 17.044 | 10.457 | 27.778 |
| | | PII(D44) | 12 | 12 | 100 | 73.5 | 100 | 16.647 | 9.980 | 27.767 |
| | | PII(D60) | 12 | 12 | 100 | 73.5 | 100 | 14.762 | 9.029 | 24.134 |
| | 10/30AS | PRE | 14 | 14 | 100 | 76.8 | 100 | 3.307 | 2.344 | 4.664 |
| | | PI(D7) | 14 | 14 | 100 | 76.8 | 100 | 14.276 | 9.854 | 20.683 |
| | | PI(D14) | 14 | 14 | 100 | 76.8 | 100 | 16.527 | 12.036 | 22.693 |
| | | PI(D30) | 14 | 12 | 85.7 | 57.2 | 98.2 | 5.479 | 1.671 | 17.963 |
| | | PII(D37) | 13 | 13 | 100 | 75.3 | 100 | 13.042 | 9.511 | 17.883 |
| | | PII(D44) | 13 | 13 | 100 | 75.3 | 100 | 12.104 | 8.706 | 16.828 |
| | | PII(D60) | 13 | 13 | 100 | 75.3 | 100 | 11.461 | 8.396 | 15.647 |
| | SALINE | PRE | 30 | 29 | 96.7 | 82.8 | 99.9 | 1.779 | 1.171 | 2.704 |
| | | PI(D7) | 30 | 29 | 96.7 | 82.8 | 99.9 | 1.831 | 1.198 | 2.797 |
| | | PI(D14) | 30 | 29 | 96.7 | 82.8 | 99.9 | 1.968 | 1.295 | 2.989 |
| | | PI(D30) | 30 | 28 | 93.3 | 77.9 | 99.2 | 1.705 | 1.055 | 2.757 |
| | | PII(D37) | 24 | 23 | 95.8 | 78.9 | 99.9 | 1.932 | 1.159 | 3.220 |
| | | PII(D44) | 23 | 22 | 95.7 | 78.1 | 99.9 | 1.929 | 1.133 | 3.286 |
| | | PII(D60) | 24 | 23 | 95.8 | 78.9 | 99.9 | 2.001 | 1.185 | 3.378 |

| | | | | | | | | | | |
|---|---|---|---|---|---|---|---|---|---|---|
| 5/10 = 5µg CPS5-TT, 5µg CPS8-TT, 10µg ClfA, 10µg α-toxoid 5/10AS = 5µg CPS5-TT, 5µg CPS8-TT, 10µg ClfA, 10µg α-toxoid adjuvanted with AS03B 10/30 = 10µg CPS5-TT, 10µg CPS8-TT, 30µg ClfA, 30µg α-toxoid 10/30AS = 10µg CPS5-TT, 10µg CPS8-TT, 30µg ClfA, 30µg α-toxoid adjuvanted with AS03B SALINE = pooling of SALINE1 and SALINE2 GMC = geometric mean antibody concentration calculated on all subjects N = number of subjects with available results n/% = number/percentage of subjects with concentration within the specified range 95% Cl = 95% confidence interval; LL = Lower Limit, UL = Upper Limit PRE = pre dose 1 PI(D7) = 7 days post dose 1 PI(D14) = 14 days post dose 1 PI(D30) = 30 days post dose 1 (blood sample taken at Visits 5 or 6) PII(D37) = 7 days post dose 2 PII(D44) = 14 days post dose 2 PII(D60) = 30 days post dose 2 | | | | | | | | | | |

## Claims

1. An immunogenic composition comprising
i. a *Staphylococcus aureus* Type 5 capsular saccharide conjugated to a carrier protein to form a *S. aureus* Type 5 capsular saccharide conjugate, wherein the *S. aureus* Type 5 capsular saccharide conjugate is administered at a saccharide dose of 3-50 µg, 5-25 µg, 3-20 µg, 3-12 µg, 5-10 µg, 7-20 µg, 7-15 µg or 8-12 µg, and
ii. a *S. aureus* Type 8 capsular saccharide conjugated to a carrier protein to form a *S. aureus* Type 8 capsular saccharide conjugate, wherein the *S. aureus* Type 8 capsular saccharide conjugate is administered at a saccharide dose of 3-50 µg, 5-25 µg, 3-20 µg, 3-12 µg, 5-10 µg, 7-20 µg, 7-15 µg or 8-12 µg, and
iii. a ClfA protein or fragment thereof wherein the ClfA protein or fragment thereof is at least 90% identical to the polypeptide sequence of any one of SEQ ID NO: 3-12 or 15-18 along the full length thereof, and
iv. an alpha toxoid
for use in treatment or prevention of *Staphylococcus aureus* infection in which a human patient is immunised with a single dose of the immunogenic composition.

2. The immunogenic composition for use in treatment or prevention of *S. aureus* infection of claim 1 wherein the *S. aureus* Type 5 capsular saccharide has a molecular weight of over 25kDa, 40kDa or 50kDa or between 25-300kDa, 50-250kDa, 70-150kDa, 25-125kDa, 90-125kDa, 30-100kDa, 35-75KDa or 40-70kDa.

3. The immunogenic composition for use in treatment or prevention of *S. aureus* infection of claim 1 or claim 2 wherein the *S. aureus* Type 8 capsular saccharide has a molecular weight of over 25kDa, 40kDa or 50kDa or between 25-300kDa, 50-250kDa, 70-150kDa, 25-125kDa, 90-125kDa, 30-100kDa, 35-75KDa or 40-70kDa.

4. The immunogenic composition for use in treatment or prevention of *S. aureus* infection of any one of claims 1-3 wherein the *S. aureus* Type 5 capsular saccharide and /or the *S. aureus* Type 8 capsular saccharide is 50-100% or 75-100% O-acetylated.

5. The immunogenic composition for use in treatment or prevention of *S. aureus* infection of any one of claims 1-4 wherein the ratio of polysaccharide to protein in the *S. aureus* Type 5 capsular saccharide conjugate is between 1:5 and 5:1 (w:w) or between 1:2 and 2:1 (w/w), 1:2 to 1:5 (w/w) or 1:1 and 1:2 (w/w).

6. The immunogenic composition for use in treatment or prevention of *S. aureus* infection of any one of claims 1-5 wherein the ratio of polysaccharide to protein in the *S. aureus* Type 8 capsular saccharide conjugate is between 1:5 and 5:1 (w:w) or between 1:2 and 2:1 (w/w), 1:2 to 1:5 (w/w) or 1:1 and 1:2 (w/w).

7. The immunogenic composition for use in treatment or prevention of *S. aureus* infection of ay one of claims 1-6 wherein the same saccharide dose of *S. aureus* Type 5 capsular saccharide and *S. aureus* Type 8 capsular saccharide is present in the immunogenic composition.

8. The immunogenic composition for use in treatment or prevention of *S. aureus* infection of any one of claims 1-7 wherein the ClfA protein or fragment thereof contains an amino acid substitution which reduces the ability of ClfA to bind to fibrinogen.

9. The immunogenic composition for use in treatment or prevention of *S. aureus* infection of claim 8 wherein the ClfA protein or fragment thereof contains an amino acid substitution at at least one of amino acids Ala254, Tyr256, Pro336, Tyr338, Ile387, Lys389, Tyr474, Glu526 or Val527.

10. The immunogenic composition for use in treatment or prevention of S. aureus infection of any one of claims 1-9 wherein the alpha toxoid has an amino acid sequence at least 90% identical to SEQ ID NO: 1-2.

11. The immunogenic composition for use in treatment or prevention of *S. aureus* infection of any one of claims 1-10 wherein the single dose of the immunogenic composition is administered 5-60, 6-40, 7-30 or 7-15 days before a planned hospital procedure.

12. An immunogenic composition comprising a *S. aureus* Type 5 capsular saccharide conjugated to a carrier protein, a *S. aureus* Type 8 capsular saccharide conjugated to a carrier protein, a ClfA protein or fragment thereof wherein the ClfA protein or fragment thereof is at least 90% identical to the polypeptide sequence of any one of SEQ ID NO:3-12 or 15-18 along the full length thereof and an alpha toxoid wherein the alpha toxoid is present in the immunogenic composition at a dose of 5-50, 10-30, 5-15 or 20-40 µg.

13. A process for making the immunogenic composition of claims 1-12 comprising the steps of a) conjugating a *S. aureus* Type 5 capsular saccharide to a carrier protein to form a *S. aureus* Type 5 capsular saccharide conjugate, b) conjugating a *S. aureus* Type 8 capsular saccharide conjugated to a carrier protein to form a *S. aureus* Type 8 capsular saccharide conjugate, and c) combining the *S. aureus* Type 5 capsular saccharide conjugate, the *S. aureus* Type 8 capsular saccharide conjugate, a ClfA protein or fragment thereof wherein the ClfA protein or fragment thereof is at least 90% identical to the polypeptide sequence of any one of SEQ ID NO: 3-12 or 15-18 along the full length thereof and an alpha toxoid to form the immunogenic composition.

## Patentansprüche

1. Immunogene Zusammensetzung, umfassend
i. ein *Staphylococcus aureus-*Typ 5-Kapselsaccharid, konjugiert an ein Trägerprotein unter Bildung eines *S. aureus-*Typ 5-Kapselsaccharidkonjugats, wobei das *S. aureus-*Typ 5-Kapselsaccharidkonjugat bei einer Sacchariddosis von 3-50 µg, 5-25 µg, 3-20 µg, 3-12 µg, 5-10 µg, 7-20 µg, 7-15 µg oder 8-12 µg verabreicht wird, und
ii. ein *S. aureus-*Typ 8-Kapselsaccharid, konjugiert an ein Trägerprotein unter Bildung eines *S. aureus-*Typ 8-Kapselsaccharidkonjugats, wobei das *S. aureus-*Typ 8-Kapselsaccharidkonjugat bei einer Sacchariddosis von 3-50 µg, 5-25 µg, 3-20 µg, 3-12 µg, 5-10 µg, 7-20 µg, 7-15 µg oder 8-12 µg verabreicht wird, und
iii. ein ClfA-Protein oder Fragment davon, wobei das ClfA-Protein oder Fragment davon zu wenigstens 90% identisch zu der Polypeptidsequenz nach irgendeiner der SEQ ID NO: 3-12 oder 15-18 entlang der gesamten Länge davon ist, und
iv. ein Alpha-Toxoid,
zur Verwendung bei der Behandlung oder Prävention von *Staphylococcus aureus*-Infektion, bei der ein menschlicher Patient mit einer einzelnen Dosis der immunogenen Zusammensetzung immunisiert wird.

2. Immunogene Zusammensetzung zur Verwendung bei der Behandlung oder Prävention von *S. aureus*-Infektion nach Anspruch 1, wobei das *S. aureus-*Typ 5-Kapselsaccharid ein Molekulargewicht von über 25 kDa, 40 kDa oder 50 kDa oder zwischen 25-300 kDa, 50-250 kDa, 70-150 kDa, 25-125 kDa, 90-125 kDa, 30-100 kDa, 35-75 kDa oder 40-70 kDa hat.

3. Immunogene Zusammensetzung zur Verwendung bei der Behandlung oder Prävention von *S. aureus*-Infektion nach Anspruch 1 oder Anspruch 2, wobei das *S. aureus-*Typ 8-Kapselsaccharid ein Molekulargewicht von über 25 kDa, 40 kDa oder 50 kDa oder zwischen 25-300 kDa, 50-250 kDa, 70-150 kDa, 25-125 kDa, 90-125 kDa, 30-100 kDa, 35-75 kDa oder 40-70 kDa hat.

4. Immunogene Zusammensetzung zur Verwendung bei der Behandlung oder Prävention von *S. aureus*-Infektion nach irgendeinem der Ansprüche 1-3, wobei das *S. aureus-*Typ 5-Kapselsaccharid und/oder das *S. aureus-*Typ 8-Kapselsaccharid zu 50-100% oder zu 75-100% O-acetyliert ist.

5. Immunogene Zusammensetzung zur Verwendung bei der Behandlung oder Prävention von *S. aureus*-Infektion nach irgendeinem der Ansprüche 1-4, wobei das Verhältnis von Polysaccharid zu Protein in dem *S. aureus-*Typ 5-Kapselsaccharidkonjugat zwischen 1:5 und 5:1 (w:w) oder zwischen 1:2 und 2:1 (w/w), 1:2 bis 1:5 (w/w) oder 1:1 und 1:2 (w/w) beträgt.

6. Immunogene Zusammensetzung zur Verwendung bei der Behandlung oder Prävention von *S. aureus-*Infektion nach irgendeinem der Ansprüche 1-5, wobei das Verhältnis von Polysaccharid zu Protein in dem *S. aureus-*Typ 8-Kapselsaccharidkonjugat zwischen 1:5 und 5:1 (w:w) oder zwischen 1:2 und 2:1 (w/w), 1:2 bis 1:5 (w/w) oder 1:1 und 1:2 (w/w) beträgt.

7. Immunogene Zusammensetzung zur Verwendung bei der Behandlung oder Prävention von *S. aureus*-Infektion nach irgendeinem der Ansprüche 1-6, wobei die gleiche Sacchariddosis von *S. aureus-*Typ 5-Kapselsaccharid und *S. aureus-*Typ 8-Kapselsaccharid in der immunogenen Zusammensetzung vorliegt.

8. Immunogene Zusammensetzung zur Verwendung bei der Behandlung oder Prävention von *S*. *aureus*-Infektion nach irgendeinem der Ansprüche 1-7, wobei das ClfA-Protein oder Fragment davon eine Aminosäuresubstitution enthält, die die Fähigkeit von ClfA zur Bindung an Fibrinogen reduziert.

9. Immunogene Zusammensetzung zur Verwendung bei der Behandlung oder Prävention von *S. aureus*-Infektion nach Anspruch 8, wobei das ClfA-Protein oder Fragment davon eine Aminosäuresubstitution an wenigstens einer der Aminosäuren Ala254, Tyr256, Pro336, Tyr338, Ile387, Lys389, Tyr474, Glu526 oder Val527 enthält.

10. Immunogene Zusammensetzung zur Verwendung bei der Behandlung oder Prävention von *S. aureus*-Infektion nach irgendeinem der Ansprüche 1-9, wobei das Alpha-Toxoid eine Aminosäuresequenz aufweist, die zu wenigstens 90% identisch zu SEQ ID NO: 1-2 ist.

11. Immunogene Zusammensetzung zur Verwendung bei der Behandlung oder Prävention von *S. aureus-*Infektion nach irgendeinem der Ansprüche 1-10, wobei die einzelne Dosis der immunogenen Zusammensetzung 5-60, 6-40, 7-30 oder 7-15 Tage vor einem geplanten Krankenhausverfahren verabreicht wird.

12. Immunogene Zusammensetzung, umfassend ein *S. aureus-*Typ 5-Kapselsaccharid, konjugiert an ein Trägerprotein, ein *S. aureus-*Typ 8-Kapselsaccharid, konjugiert an ein Trägerprotein, ein ClfA-Protein oder Fragment davon, wobei das ClfA-Protein oder Fragment davon zu wenigstens 90% identisch zu der Polypeptidsequenz nach irgendeiner der SEQ ID NO: 3-12 oder 15-18 entlang der gesamten Länge davon ist, und ein Alpha-Toxoid, wobei das Alpha-Toxoid in der immunogenen Zusammensetzung in einer Dosis von 5-50, 10-30, 5-15 oder 20-40 µg vorliegt.

13. Verfahren zum Herstellen der immunogenen Zusammensetzung nach den Ansprüchen 1-12, umfassend die Schritte a) des Konjugierens eines *S. aureus-*Typ 5-Kapselsaccharids an ein Trägerprotein unter Bildung eines *S. aureus-*Typ 5-Kapselsaccharidkonjugats, b) des Konjugierens eines *S. aureus-*Typ 8-Kapselsaccharids, konjugiert an ein Trägerprotein, unter Bildung eines *S. aureus-*Typ 8-Kapselsaccharidkonjugats, und c) des Kombinierens des *S. aureus-*Typ 5-Kapselsaccharidkonjugats, des *S. aureus-*Typ 8-Kapselsaccharidkonjugats, eines ClfA-Proteins oder Fragments davon, wobei das ClfA-Protein oder Fragment davon zu wenigstens 90% identisch zu der Polypeptidsequenz nach irgendeiner der SEQ ID NO: 3-12 oder 15-18 entlang der gesamten Länge davon ist, und eines Alpha-Toxoids unter Bildung der immunogenen Zusammensetzung.

## Revendications

1. Composition immunogène comprenant
i. un saccharide capsulaire de type 5 de *Staphylococcus aureus* conjugué à une protéine porteuse pour former un conjugué de saccharide capsulaire de type 5 de S. *aureus,* dans laquelle le conjugué de saccharide capsulaire de type 5 de S. *aureus* est administré à une dose de saccharide de 3 à 50 µg, 5 à 25 µg, 3 à 20 µg, 3 à 12 µg, 5 à 10 µg, 7 à 20 µg, 7 à 15 µg ou 8 à 12 µg, et
ii. un saccharide capsulaire de type 8 de *S. aureus* conjugué à une protéine porteuse pour former un conjugué de saccharide capsulaire de type 8 de *S. aureus,* dans laquelle le conjugué de saccharide capsulaire de type 8 de *S. aureus* est administré à une dose de saccharide de 3 à 50 µg, 5 à 25 µg, 3 à 20 µg, 3 à 12 µg, 5 à 10 µg, 7 à 20 µg, 7 à 15 µg ou 8 à 12 µg, et
iii. une protéine ClfA ou un fragment de celle-ci, dans laquelle la protéine ClfA ou le fragment de celle-ci est au moins identique à 90 % à la séquence de polypeptide de l'une quelconque de SEQ ID N° 3 à 12 ou 15 à 18 sur toute sa longueur, et
iv. un toxoïde alpha
pour une utilisation dans le traitement ou la prévention d'une infection par *Staphylococcus aureus* dans laquelle un patient humain est immunisé avec une dose unique de la composition immunogène.

2. Composition immunogène pour une utilisation dans le traitement ou la prévention d'une infection par *S. aureus* selon la revendication 1, dans laquelle le saccharide capsulaire de type 5 de *S. aureus* a une masse moléculaire au-delà de 25 kDa, 40 kDa ou 50 kDa ou entre 25 et 300 kDa, 50 et 250 kDa, 70 et 150 kDa, 25 et 125 kDa, 90 et 125 kDa, 30 et 100 kDa, 35 et 75 kDa ou 40 et 70 kDa.

3. Composition immunogène pour une utilisation dans le traitement ou la prévention d'une infection par *S. aureus* selon la revendication 1 ou la revendication 2, dans laquelle le saccharide capsulaire de type 8 de *S. aureus* a une masse moléculaire au-delà de 25 kDa, 40 kDa ou 50 kDa ou entre 25 et 300 kDa, 50 et 250 kDa, 70 et 150 kDa, 25 et 125 kDa, 90 et 125 kDa, 30 et 100 kDa, 35 et 75 kDa ou 40 et 70 kDa.

4. Composition immunogène pour une utilisation dans le traitement ou la prévention d'une infection par S. *aureus* selon l'une quelconque des revendications 1 à 3, dans laquelle le saccharide capsulaire de type 5 de *S. aureus* et/ou le saccharide capsulaire de type 8 de S. *aureus* sont O-acétylés à 50-100 % ou 75-100 %.

5. Composition immunogène pour une utilisation dans le traitement ou la prévention d'une infection par *S. aureus* selon l'une quelconque des revendications 1 à 4, dans laquelle le rapport polysaccharide sur protéine dans le conjugué de saccharide capsulaire de type 5 de *S. aureus* est entre 1:5 et 5:1 (p/p) ou entre 1:2 et 2:1 (p/p), 1:2 à 1:5 (p/p) ou 1:1 et 1:2 (p/p).

6. Composition immunogène pour une utilisation dans le traitement ou la prévention d'une infection par *S. aureus* selon l'une quelconque des revendications 1 à 5, dans laquelle le rapport polysaccharide sur protéine dans le conjugué de saccharide capsulaire de type 8 de *S. aureus* est entre 1:5 et 5:1 (p/p) ou entre 1:2 et 2:1 (p/p), 1:2 à 1:5 (p/p) ou 1:1 et 1:2 (p/p).

7. Composition immunogène pour une utilisation dans le traitement ou la prévention d'une infection par *S. aureus* selon l'une quelconque des revendications 1 à 6, dans laquelle la même dose de saccharide de saccharide capsulaire de type 5 de *S. aureus* et de saccharide capsulaire de type 8 de *S. aureus* est présente dans la composition immunogène.

8. Composition immunogène pour une utilisation dans le traitement ou la prévention d'une infection par *S. aureus* selon l'une quelconque des revendications 1 à 7, dans laquelle la protéine ClfA ou le fragment de celle-ci contient une substitution d'acide aminé qui réduit l'aptitude de ClfA à se lier à un fibrinogène.

9. Composition immunogène pour une utilisation dans le traitement ou la prévention d'une infection par *S. aureus* selon la revendication 8, dans laquelle la protéine ClfA ou le fragment de celle-ci contient une substitution d'acide aminé en au moins l'un des acides aminés Ala254, Tyr256, Pro336, Tyr338, Ile387, Lys389, Tyr474, Glu526 ou Val527.

10. Composition immunogène pour une utilisation dans le traitement ou la prévention d'une infection par *S. aureus* selon l'une quelconque des revendications 1 à 9, dans laquelle le toxoïde alpha a une séquence d'acides aminés au moins identique à 90 % à SEQ ID N° 1 à 2.

11. Composition immunogène pour une utilisation dans le traitement ou la prévention d'une infection par *S. aureus* selon l'une quelconque des revendications 1 à 10, dans laquelle la dose unique de la composition immunogène est administrée 5 à 60, 6 à 40, 7 à 30 ou 7 à 15 jours avant une procédure hospitalière programmée.

12. Composition immunogène comprenant un saccharide capsulaire de type 5 de *S. aureus* conjugué à une protéine porteuse, un saccharide capsulaire de type 8 de *S. aureus* conjugué à une protéine porteuse, une protéine ClfA ou un fragment de celle-ci dans laquelle la protéine ClfA ou le fragment de celle-ci est au moins identique à 90 % à la séquence de polypeptide de l'une quelconque de SEQ ID N° 3 à 12 ou 15 à 18 sur toute sa longueur et un toxoïde alpha, dans laquelle le toxoïde alpha est présent dans la composition immunogène à une dose de 5 à 50, 10 à 30, 5 à 15 ou 20 à 40 µg.

13. Procédé de préparation de la composition immunogène des revendications 1 à 12 comprenant les étapes de a) conjugaison d'un saccharide capsulaire de type 5 de *S. aureus* à une protéine porteuse pour former un conjugué de saccharide capsulaire de type 5 de *S. aureus,* b) conjugaison d'un saccharide capsulaire de type 8 de *S. aureus* conjugué à une protéine porteuse pour former un conjugué de saccharide capsulaire de type 8 de *S. aureus,* et c) combinaison du conjugué de saccharide capsulaire de type 5 de *S. aureus,* du conjugué de saccharide capsulaire type 8 de *S. aureus,* d'une protéine ClfA ou d'un fragment de celle-ci, dans lequel la protéine ClfA ou le fragment de celle-ci est au moins identique à 90 % à la séquence de polypeptide de l'une quelconque de SEQ ID N° 3 à 12 ou 15 à 18 sur toute sa longueur, et d'un toxoïde alpha pour former la composition immunogène.
